(19) Europäisches Patentamt
European Patent Office
Office européen des brevets

(11) Publication number: **0 105 227**
**A1**

(12) **EUROPEAN PATENT APPLICATION**

(21) Application number: **83108896.8**

(22) Date of filing: **08.09.83**

(51) Int. Cl.³: **C 07 D 487/04**
// (C07D487/04, 209/00, 205/00)

(30) Priority: **10.09.82 JP 156794/82**

(43) Date of publication of application: **11.04.84**
**Bulletin 84/15**

(84) Designated Contracting States: **AT BE CH DE FR GB IT LI**

(71) Applicant: **SANRAKU-OCEAN CO., LTD., 15-1, Kyobashi 1-chome Chuo-ku, Tokyo (JP)**

(72) Inventor: **Yoshioka, Takeo, Green Haitsu 3-3102 Kamitsuchidana 1959, Ayase-shi Kanagawa-ken (JP)**
Inventor: **Watanabe, Azuma, 2-23-1, Nagata Kita Minami-ku, Yokohama-shi Kanagawa-ken (JP)**
Inventor: **Shimauchi, Yasutaka, 1-101-13, Yurigaoka Ninomiya-cho, Naka-gun Kanagawa-ken (JP)**
Inventor: **Fukagawa, Yasuo, 9-2, Imaizumidai 3-chome, Kamakura-shi Kanagawa-ken (JP)**
Inventor: **Ishikura, Tomoyuki, 22-32, Kowada 1-chome, Chigasaki-shi Kanagawa-ken (JP)**

(74) Representative: **Kraus, Walter, Dr. et al, Patentanwälte Dres. Kraus & Weisert Irmgardstrasse 15, D-8000 München 71 (DE)**

(54) **Process for producing beta-lactam compounds, and intermediates useful for their production.**

(57) A process for producing a beta-lactam compound of the general formula

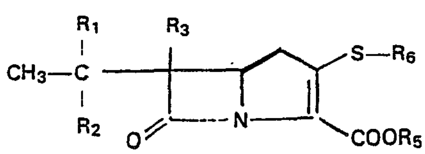

(III)

wherein R represents a hydrogen atom or a methyl group, $R_2$ represents a hydrogen atom, a hydroxyl group which may be protected or a hydroxymethyl group which may be protected, $R_3$ represents a hydrogen atom, or together with $R_1$ forms a single bond, $R_5$ represents an ester residue and $R_6$ represents a hydrogen atom or a monovalent organic group, which comprises reacting a compound of the general formula

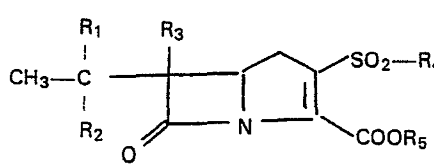

(I)

wherein $R_1$, $R_2$, $R_3$ and $R_5$ are as defined above, $R_4$ represents $-CH_2-CH_2-$ or $-CH=CH-$, and Y represents a protected amino group, with a thiol compound of the formula

$$R_6-SH \qquad (II)$$

or its reactive derivative; and noble intermediate useful for the above process.

EP 0 105 227 A1

0105227

This invention relates to a novel process for producing 7-oxo-1-azabicylco[3.2.0]hept-2-ene-2-carboxylic acid derivatives and synthetic intermediates thereof. More specifically, this invention relates to a versatile process for producing beta-lactam compounds of the following general formula which have excellent antibacterial activity and are useful as antibacterial agents.

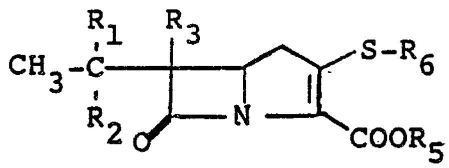

(III)

wherein $R_1$ represents a hydrogen atom or a methyl group, $R_2$ represents a hydrogen atom, a hydroxyl group which may be protected, or a hydroxymethyl group which may be protected, $R_3$ represents a hydrogen atom or together with $R_1$ forms a single bond, $R_5$ represents an ester residue, and $R_6$ represents a hydrogen atom or a monovalent organic group.

As a versatile process for producing a group of beta-lactam compounds included within the above formula (III), the present inventors previously proposed a process for producing beta-lactam compounds of the following formula

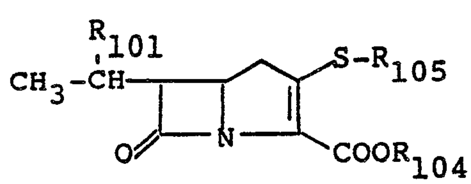

(I-2)

wherein $R_{101}$ represents a hydrogen atom, a methyl group or a hydroxyl group, $R_{104}$ represents a hydrogen atom or an ester

residue, and $R_{105}$ represents a hydrogen atom or monovalent organic group,

which comprises reacting a compound of the following general formula

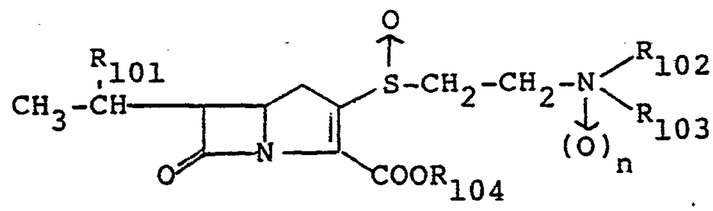

(IV)

wherein $R_{101}$ and $R_{104}$ are as defined, each of $R_{102}$ and $R_{103}$ represents a hydrogen atom, an alkyl group or an N-protecting group, and n is 0 or 1,

or its salt with a thiol compound of the formula

$$R_{105}-SH \qquad (V)$$

wherein $R_{105}$ is as defined,

or its reactive derivative (see European Laid-Open Patent Publication No. 11173A).

On further investigations, the present inventors have now found that when a sulfone compound of the following general formula (I)

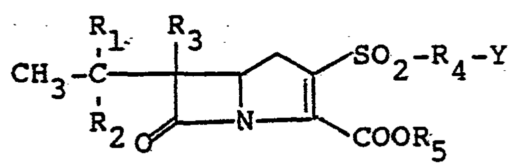

(I)

wherein $R_4$ represents $-CH_2-CH_2-$ or $-CH=CH-$, Y represents a protected amino group, and $R_1$, $R_2$, $R_3$, $R_5$ and $R_6$ are as defined above,

is used instead of the above S-oxide compound of formula (IV), the same substitution reaction proceeds to produce the compound of formula (III).

Thus, according to this invention, there is provided a process for producing a beta-lactam compound of the general formula

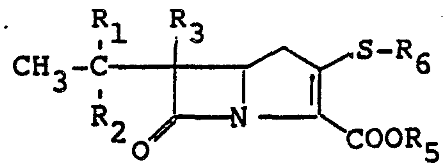

(III)

wherein $R_1$ represents a hydrogen atom or a methyl group, $R_2$ represents a hydrogen atom, a hydroxyl group which may be protected or a hydroxymethyl group which may be protected, $R_3$ represents a hydrogen atom, or together with $R_1$ forms a single bond, $R_5$ represents an ester residue and $R_6$ represents a hydrogen atom or a monovalent organic group,

which comprises reacting a compound of the general formula

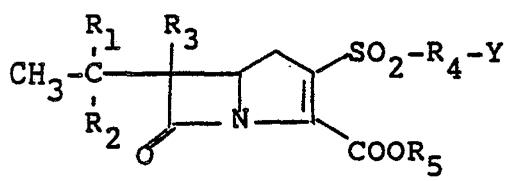

(I)

wherein $R_1$, $R_2$, $R_3$ and $R_5$ are as defined above, $R_4$ represents $-CH_2-CH_2-$ or $-CH=CH-$, and Y represents a protected amino group,

with a thiol compound of the formula

$R_6$-SH (II)

or its reactive derivative.

In the present application, the term "lower" which qualifies a group or compound means that the group or compound so qualified has not more than 6, preferably not more than 4, carbon atoms.

In formula (I), the "hydroxyl group which may be protected" and the "hydroxymethyl group which may be protected" which are represented by $R_2$ denote a hydroxyl and a hydroxymethyl group in which the hydrogen atom of the hydroxyl group (-OH) is substituted by any of various protective groups for the hydroxyl group which are commonly used in the art. Typical examples of the protective groups include lower alkyl groups such as methyl, ethyl, propyl and butyl; unsubstituted or substituted aralkyl groups such as benzyl and p-nitrobenyl; lower alkoxyalkyl groups such as methoxymethyl and ethoxymethyl; tetrahydropyranyl and tetrahydrofuranyl; acyl groups such as formyl, acetyl, propionyl, butyryl, p-tolyl, cyclohexylcarbonyl, chloroacetyl, dichloroacetyl, trichloroacetyl, trifluoroacetyl, benzoyl, furoyl, phenacyl and acetonyl; alkoxycarbonyl groups such as methoxyacetyl, methoxycarbonyl, ethoxycarbonyl, ethoxycarbonyl, butoxycarbonyl, benzyloxycarbonyl, phenoxycarbonyl and p-nitrobenzyloxycarbonyl; and organic sulfonyl groups such as mesyl, tosyl, dimethoxyphosphoryl, diethoxyphosphoryl, methoxysulfonyl and ethoxysulfonyl.

The "protected amino group" represented by Y in formula (I) denotes an amino group ($-NH_2$) in which one or two of the two hydrogen atoms are substituted by amino protecting groups which are known in the art. Typical examples of such amino protecting groups are given below.

0105227

(a) Substituted or unsubstituted aralkyl groups such as benzyl, p-nitrobenzyl, m-chlorobenzyl, p-methoxybenzyl, benzhydryl and trityl.

(b) Groups of the formula $-COR_9$ [wherein $R_9$ represents a hydrogen atom, an alkyl group (such as methyl, ethyl, n-propyl, isopropyl, n-butyl and tert-butyl), a cycloalkyl group (such as cyclopentyl and cyclohexyl), a substituted or unsubstituted aryl group (such as a substituted or unsubstituted phenyl group, for example phenyl, p-methoxyphenyl and p-nitrophenyl), or a substituted or unsubstituted aralkyl group (such as a substituted or unsubstituted benzyl group, for example benzyl, p-nitrobenzyl, m-chlorobenzyl, p-bromobenzyl and p-methoxybenzyl)], such as formyl, acetyl, propionyl, benzoyl, p-nitrobenzoyl, m-chlorobenzoyl, p-bromobenzoyl, phenylacetyl, p-methoxyphenylacetyl and p-nitrophenylacetyl; and groups of the formula

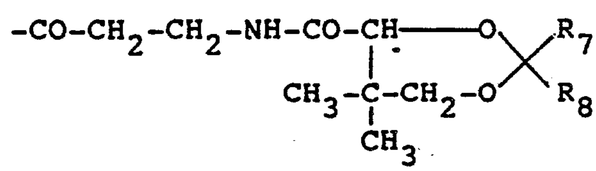

wherein each of $R_7$ and $R_8$ represents a hydrogen atom, a lower alkyl group or a phenyl group, or $R_7$ and $R_8$ together represent an alkylene group having 4 or 5 carbon atoms, for example $R_7$ and $R_8$ individually reprent a hydrogen atom and a hydrogen atom, a hydrogen atom and a methyl group, a hydrogen atom and methyl group, a hydrogen atom and a propyl group, a hydrogen atom and a phenyl group, a methyl group and a phenyl group, a methyl group and an ethyl group, or a phenyl group and a phenyl group, or together represent a tetramethylene or pentamethylene group.

(c) Groups of the formula $-COOR_{10}$ [wherein $R_{10}$ represents an alkyl group (for example, a lower

alkyl group such as methyl, ethyl, n-propyl, isopropyl, n-butyl and tert-butyl), a cycloalkyl group (such as cyclopentyl or cyclohexyl) or a substituted or unsubstituted aralkyl group (for example, a substituted or unsubstituted benzyl group such as benzyl, p-nitrobenzyl, m-chlorobenzyl, p-bromobenzyl and p-methoxybenzyl)], such as methoxycarbonyl, ethoxycarbonyl, tert-butoxycarbonyl, benzyloxycarbonyl and p-nitrobenzyloxycarbonyl.

(d) Groups of the formula $-SO_2-R_{11}$ (wherein $R_{11}$ represents a lower alkyl group such as methyl or a substituted or unsubstituted aryl group such as phenyl or tolyl), such as mesyl, benzenesulfonyl and tosyl.

(e) Groups of the formula

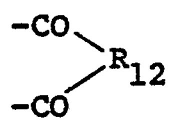

wherein $R_{12}$ represents a lower alkylene group, a lower alkenylene group or a phenylene group, such as succinyl, maleinyl, and phthalyl.

(f) Trimethylsilyl, and 4,4-dimethyl-1-oxo-2-hexen-3-yl.

The "ester residue" represented by $R_5$ in formula (I) and the "monovalent organic group" represented by $R_6$ in formulae (II) and (III) denote a hydrocarbon group which may contain at least one hetero atom in the carbon main chain or carboxylic skeleton and/or the side chain or in a substituent. The hydrocarbon group may be any of saturated or unsaturated aliphatic groups, alicyclic groups, alicyclic-aliphatic groups, aromatic groups, aromatic-aliphatic groups, heterocyclic groups and heterocyclic-aliphatic groups, which suitably have up to 30 carbon atoms, preferably up to 25 carbon atoms, more preferably up to 20 carbon atoms.

They include, for example, the following groups.

(1) Substituted or unsubstituted alkyl, alkenyl or alkynyl groups.

(2) Cycloalkyl groups.

(3) Cycloalkyl-alkyl groups.

(4) Substituted or unsubstituted aryl groups.

(5) Substituted or unsubstituted aralkyl groups.

(6) Heterocyclic groups.

(7) Heterocyclic-alkyl groups.

These groups will be exemplified more specifically below. It should be understood however that the following description of these groups is merely illustrative, and does not in any way limit the scope of this invention.

(1) Substituted or unsubstituted alkyl, alkenyl or alkynyl groups.

(1-1) Examples of suitable alkyl groups are those having 1 to 14 carbon atoms, preferably 1 to 12 carbon atoms, more preferably 1 to 10 carbon atoms. They may be either linear or branched. Typical examples of such alkyl groups include methyl, ethyl, n- or iso-propyl, n-, iso-, sec- or tert-butyl, n-pentyl, iso-amyl, n-hexyl, n-heptyl, n-octyl, 2-ethylhexyl, n-nonyl, n-decyl, n-undecyl and n-dodecyl.

(1-2) The alkenyl groups may be linear or branched, and have 2 to 10 carbon atoms, preferably 2 to 6 carbon atoms. Lower alkenyl groups are especially suitable. Examples include vinyl, allyl, 2-butenyl and 3-methyl-2-butenyl.

(1-3) The alkynyl groups are unsaturated aliphatic hydrocarbon groups having a triple bond in the carbon chain, and in this invention, lower alkynyl groups are preferred. Examples include propargyl and

ethynyl.

(1-4) The alkyl, alkenyl and alkynyl groups mentioned in (1-1), (1-2) and (1-3) , especially the alkyl groups, may be substituted by a group selected from the class consisting of halogen atoms, a hydroxyl group, lower alkoxy groups, lower alkylthio groups, cycloalkyloxy groups having 3 to 12 carbon atoms, cycloalkylthio groups having 3 to 12 carbon atoms, aryloxy groups (the aryl moiety is preferably a phenyl group and may be substituted by a halogen atom, a lower alkoxy group, a lower haloalkyl group or a nitro group), arylthio groups (the aryl moiety is preferably a phenyl group, and may be substituted by a halogen atom, a lower alkoxy group, a lower haloalkyl group or a nitro group), di-($C_{1-10}$ alkyl)-amino groups, $C_{2-16}$ acylamino groups, aryloylimino groups, $C_{2-16}$ acyloxy groups, $C_{2-16}$ acylthio groups, $C_{2-16}$ acyl groups, $C_{2-14}$ alkoxycarbonyl groups (the alkoxy moiety may be substituted by a halogen atom), and $C_{8-24}$ aralkyloxycarbonyl groups (the aralkyl moiety may be substituted by a halogen atom, a lower alkoxy group, a lower haloalyl group or a nitro group).

The acyl moiety in the above "acylamino groups", "acyloxy groups", "acylthio groups" and "acyl groups" means a group of the formula $R_9CO$- resulting from the removal of OH from an organic carboxylic acid, and the group $R_9$ may, for example, be an alkyl group, a cycloalkyl group, an aryl group or an aralkyl group, each of which may be substituted by a halogen atom, a lower alkoxy group, a lower haloalkyl group, a nitro group, a di(lower alkyl)-amino group, etc.

Typical examples of alkyl groups substituted by such groups include

2,2,2-trichloroethyl,
2,2,2-trifluoroethyl,
hydroxyethyl,
2-hydroxypropyl,
methoxymethyl,
ethoxymethyl,
isopropoxymethyl,
ethoxyethyl,
ethoxypropyl,
methylthiomethyl,
ethylthiomethyl,
ethylthioethyl,
methylthiopropyl,
isopropylthioethyl,
cyclohexyloxymethyl,
cyclohexylthiomethyl,
phenoxymethyl,
phenoxyethyl,
p-chlorophenoxymethyl,
p-methoxyphenoxymethyl,
p-trifluoromethylphenoxymethyl,
p-nitrophenoxymethyl,
phenylthiomethyl,
p-methoxyphenylthiomethyl,
p-trifluoromethylphenylthiomethyl,
p-nitrophenylthiomethyl,
2-dimethylaminoethyl,
3-diethylaminopropyl,
acetylaminomethyl,
propionylaminomethyl,
phthaloyliminomethyl,
acetoxymethyl,
acetoxyethyl,
propionyloxymethyl,
acetoxypropyl,
pivaloyloxymethyl,

benzoyloxymethyl,
trichloroacetoxymethyl,
dimethylaminoacetoxymethyl,
p-methoxybenzoyloxymethyl,
p-nitrobenzoyloxymethyl,
phenylacetoxymethyl,
acetylthiomethyl,
acetylthioethyl,
acetonyl,
propionylmethyl,
phenacyl,
p-chlorophenacyl,
p-bromophenacyl,
p-methylphenacyl,
p-tert-butylphenacyl,
p-ethylphenacyl,
p-nitrophenacyl,
o-nitrophenacyl,
p-trifluoromethylphenacyl,
p-methoxyphenacyl,
benzoylethyl,
phenylacetonyl,
methoxycarbonylmethyl,
ethoxycarbonylmethyl,
carbamoylmethyl,
carbamoylethyl,
tert-butoxycarbonylmethyl,
tert-amyloxycarbonylmethyl,
2,2,2-trichloroethoxycarbonylmethyl,
2,2,2-tribromoethoxycarbonylmethyl,
benzyloxycarbonylmethyl,
diphenylmethoxycarbonylmethyl,
triphenylmethoxycarbonylmethyl,
p-nitrobenzyloxycarbonylmethyl,
p-nitrobenzyloxycarbonylethyl,
p-methoxybenzyloxycarbonylmethyl,

phenethyloxycarbonylmethyl, and 2-amino-2-p-nitrobenzyloxycarbonylethyl.

(2) Cycloalkyl groups

Suitable cycloalkyl groups have 3 to 15 carbon atoms, preferably 3 to 10 carbon atoms, more preferably 3 to 6 carbon atoms, and include those which contain a lower alkyl group on the ring. Typical examples include cyclopropyl, cyclobutyl, cyclopentyl, cyclohexyl, cycloheptyl, cyclooctyl, 4-methylcyclohexyl and 4-ethylcyclohexyl.

(3) Cyclkoalkyl-alkyl groups

Preferred cycloalkyl-alkyl groups are those in which the cycloalkyl moiety has the meaning described n section (2) above, and the alkyl moiety is a lower alkyl group. Suitable cycloalkyl-alkyl groups have 4 to 19 carbon atoms, preferably 4 to 14 carbon atoms, particularly 4 to 10 carbon atoms. Typical examples include cyclopentylmethyl, cyclohexylmethyl, cyclohexylethyl and cycloheptylmethyl.

(4) Substituted or unsubstituted aryl groups

(4-1) The aryl groups may be monocyclic or polycyclic, and have usually 6 to 18 carbon atoms, preferably 6 to 14 carbon atoms, more preferably 6 to 10 carbon atoms. Typical examples of the aryl group are phenyl, tolyl, xylyl and naphthyl.

(4-2) The aryl groups may have a substituent on the aromatic ring. Examples of the substituent include halogen atoms, lower alkoxy groups, aryloxy groups, lower haloalkyl groups, acyloxy groups, acylamino groups, a carboxyl group, carboxylate salt groups, lower alkoxycarbonyl groups, a hydroxyl group, and a nitro group.

Examples of aryl groups substituted by these groups include p-chlorophenyl, p-methoxyphenyl, p-trifluoromethylphenyl, p-acetyloxyphenyl, p-acetylaminophenyl, p-methoxycarbonylphenyl, p-nitrophenyl,

and 2,4-dinitrophenyl.

(5) Substituted or unsubstituted aralkyl groups

(5-1) The aralkyl groups may be monocyclic or polycyclic, and the alkyl moiety is preferably lower. The aralkyl groups may have usually 7 to 25 carbon atoms, preferably 7 to 22 carbon atoms, more preferably 7 to 19 carbon atoms. Examples of such aralkyl groups include benzyl, p-tert-butylbenzyl, p-methylbenzyl, 2,4-dimethylbenzyl, 2,4,6-trimethylbenzyl, benzhydryl, 1,1-diphenylethyl, 1,1-diphenylpropyl, 1,1-diphenylbutyl, trityl, and p-methyltrityl.

(5-2) A substituent may exist on the aromatic ring of such aralkyl groups. Examples of suitable substituents are a halogen atom, a lower alkoxy group, an aryloxy group, a lower haloalkyl group, an acyloxy group, an acylamino group, a carboxyl group, a carboxylate salt group, a lower alkoxycarbonyl group, a hydroxyl group, or a nitro group.

Typical examples of aralkyl groups substituted by these substituents include

p-chlorobenzyl,
p-bromobenzyl,
p-methoxybenzyl,
p-tert-butoxybenzyl,
3,5-bis-tert-butoxy-4-hydroxybenzyl,
m-phenoxybenzyl,
p-trifluoromethylbenzyl,
o- or p-pivaloyloxybenzyl,
p-acetoxybenzyl,
p-benzoyloxybenzyl,
p-2-ethylhexanoylbenzyl,
p-benzamidobenzyl,
p-carboxybenzyl(the carboxyl may be an alkali metal carboxylate),
p-methoxycarbonylbenzyl,

p-ethoxycarbonylbenzyl,
p-butoxycarbonylbenzyl,
p-hydroxybenzyl,
o- or p-nitrobenzyl,
p-chlorobenzhydryl,
p-methoxybenzhydryl,
p-acetoxybenzhydryl,
p-nitrobenzhydryl,
m- or p-chlorotrityl,
p-bromotrityl,
p-methoxytrityl,
p-ethoxytrityl, and
p-nitrotrityl.

(6) Heterocyclic groups

The heterocyclic groups contain 1 to 8 carbon atoms selected from nitrogen, oxygen and sulfur atoms as hetero atoms, and may have on the heterocyclic ring a substituent such as a halogen atom, a lower alkyl group, a lower alkoxy group, an aryloxy group, a lower haloalkyl group, an acyloxy group, an acylamino group, a carboxyl group, a carboxylate salt group, a lower alkoxycarbonyl group, a hydroxyl group, or a nitro group. Preferably, the heterocyclic groups are 3- to 10-membered, particularly 5- to 7-membered. Typical examples of such heterocyclic groups include 2-pyridyl, 4-pyridyl, 2-pyrimidinyl, 2-pyradinyl, 2-thienyl, 3-thienyl, 2-furyl, 3-furyl, 2-thiazolyl, 2-imidazolyl, 1,2,3-triazolyl, 1,2,4-triazolyl, 5-tetrazolyl, 2-pyrrolyl, 2-morpholino, 3-morpholino, 2-piperidinyl, 3-piperidinyl, 4-piperidinyl, 2-pyrrolydinyl, 2-pyranyl, 3-pyranyl, 4-pyranyl, 2-tetrahydrofuranyl, and 3-tetrahydrofuranyl.

(7) Heterocyclic-alkyl groups

The heterocyclic ring moiety of these groups contains 1 to 8 atoms selected from nitrogen, oxygen

and sulfur atoms as hetero atoms, and may have on the heterocyclic ring a substituent such as a halogen atom, a lower alkyl group, a lower alkoxy group, an aryloxy group, a lower haloalkyl group, an acyloxy group, an acylamino gorup, a carboxyl group, a carboxylate salt group, a lower alkoxycarbonyl group, a hydroxyl group and a nitro group. Preferably, the heterocyclic ring can be 3- to 10-membered, particularly 5- to 7-membered. Desirably, the alkyl moiety is lower. Thus, the heterocyclic-alkyl groups may generally have 2 to 25 carbon atoms, preferably 2 to 18 carbon atoms, more preferably 2 to 12 carbon atoms.

Examples of such heterocyclic-alkyl groups include

2-pyridylmethyl,
2-pyridylethyl,
3-pyridylmethyl,
3-pyridylethyl,
4-pyridylmethyl,
4-pyridylethyl,
2-pyrimidinylmethyl,
2-pyrimidinylethyl,
4-pyrimidinylmethyl,
4-pyrimidinylethyl,
5-pyrimidinylmethyl,
5-pyrimidinylethyl,
2-imidazolylmethyl,
2-imidazolylethyl,
4-imidazolylmethyl,
4-imidazolylethyl,
2-furylmethyl,
2-furylethyl,
3-furylmethyl,
3-furylethyl,
2-pyradinylmethyl,

2-pyradinylethyl,
2-thiazolylmethyl,
2-thiazolylethyl,
3-thiazolylmethyl,
3-thiazolylethyl,
4-thiazolylmethyl,
4-thiazolylethyl,
2-thienylmethyl,
2-thienylethyl,
3-thienylmethyl,
3-thienylethyl,
2-pyrrolylmethyl,
2-pyrrolylethyl,
3-pyrrolylmethyl,
3-pyrrolylethyl,
5-tetrazolylmethyl,
5-tetrazolylethyl,
4-(1,2,3)triazolylmethyl,
4-(1,2,3)triazolylethyl,
3-(1,2,4)triazolylmethyl,
3-(1,2,4)triazolylethyl,
2-morpholinomethyl,
3-morpholinomethyl,
2-morpholinoethyl
3-morpholinoethyl,
2-piperidinylmethyl,
3-piperidinylmethyl,
4-piperidinylmethyl,
2-piperidinylethyl,
3-piperidinylethyl,
4-piperidinylethyl,
2-pyrrolidinylmethyl,
3-pyrrolidinylmethyl,
2-pyrrolidinylethyl,
3-pyrrolidinylethyl,
2-piranylmethyl,

0105227

3-pyranylmethyl,
4-pyranylmethyl,
2-pyranylethyl,
3-pyranylethyl,
4-pyranylethyl,
2-tetrahydrofuranylmethyl,
3-tetrahydrofuranylmethyl,
2-tetrahydrofuranylethyl, and
3-tetrahydrofuranylethyl.

Of these, examples of preferred ester residues represented by $R_5$ include pivaloyloxymethyl, phthalidyl, methylthiomethyl, benzyl, benzhydryl, trityl, p-nitrobenzyl, 2,4-dinitrobenzyl, and p-chlorobenzyl groups. Especially preferred ester residues are those which can be easily removed by hydrolysis or hydrogenolysis.

Preferred "monovalent organic groups" represented by $R_6$ include the following.

(a) Lower alkyl groups.

(b) $C_{5-6}$ cycloalkyl groups.

(c) Substituted lower alkyl groups (the substituent being a hydroxyl group, a lower alkoxy group, a lower alkanoyl group, an amino group, a mono- or di-lower alkylamino group, a carbamoyl group, a lower alkoxycarbonyl group, a substituted or unsubstituted phenoxycarbonyl group, a substituted or unsubstituted benzoylamino group, a group of the formula

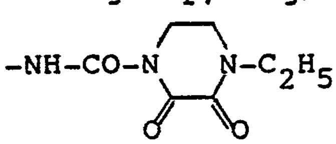

a group of the formula

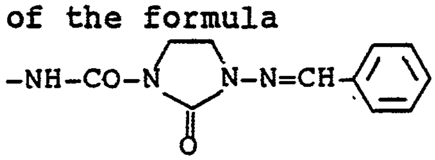

or a 5- or 6-membered heterocyclic group containing 1 or 2 N, O or S atoms).

(c) Five- or six-membered heterocyclic groups having 1 to 4 N, O or S atoms as hetero atoms, which may be substituted by a methyl or nitro group.

Advantageously, the reaction of the compound of formula (I) with the thiol compound of formula (II) or its reactive derivative in accordance with this invention is carried out usually in an inert polar solvent such as N,N-dimethylformamide (DMF), tetrahydrofuran (THF), dioxane, hexamethylphosphotriamide (HMPA), or glyme, particularly in DMF, generally at a temperature of room temperature or below, preferably about -30 to about -50°C. The reaction can be terminated usually in 15 to 60 minutes under these conditions.

When the thiol compound of formula (II) is directly used, the reaction is usually carried out in the presence of a base, but even in the absence of a base, the reaction proceeds. Examples of usable bases include sodium hydride, potassium hydride, sodium amide, potassium amide, sodium hydroxide, potassium hydroxide, sodium ethoxide, sodium methoxide, potassium butoxide, triethylamine and tripropylamine. Advantageously, the base is used in a proportion of at least 1 equivalent, preferably 1.0 to 2.0 equivalents, per mole of the thiol compound.

Instead of performing the above reaction in the presence of bases, a reactive derivative of the thiol compound of formula (II) may be used. Examples of the reactive derivative include thiolate compounds of the following formula

$$R_6-SM \qquad (II')$$

wherein M represents an alkali metal and $R_6$ is as defined hereinabove.

The amount of the thiol compound of formula

(II) or its reactive derivative used in this reaction is not critical. Generally, it is advantageously used in a proportion of at least 1.0 mole, preferably 1.1 to 1.5 moles, per mole of the compound of formula (I).

The above reaction gives the compound of formula (III). When $R_5$ is an ester residue easily removable by hydrolysis or hydrogenolysis, this compound may be subjected to saponification or hydrogenolysis, for example, in a customary manner (for example, as shown in Examples 19 to 31 given hereinbelow) to remove the group $R_5$, after it is isolated and purified as required by methods described below. This treatment gives a compound of the following formula

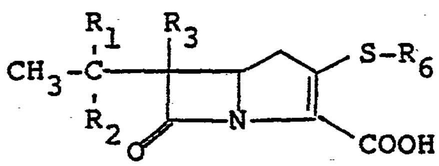

(III')

wherein $R_1$, $R_2$, $R_3$ and $R_6$ are as defined above, or its salt.

Examples of the salt of the compound of formula (III') include its inorganic salts such as sodium, potassium, magnesium, calcium and ammonium salts, and its salts with organic bases such as monoethylamine, dimethylamine, triethylamine, monoethanolamine, diethanolamine, benzathine and procaine.

The isolation and purification of the compound of formula (III) can be effected, for example, by diluting the reaction mixture with a water-immiscible inert solvent such as benzene, toluene, methylene chloride or ethyl acetate, washing the mixture first with a phosphate buffer (pH 8.7) or an aqueous solution of sodium bicarbonate and then with a phosphate

buffer (pH 6.8), drying the solvent layer over anhydrous sodium sulfate, evaporating the solvent under reduced pressure, dissolving the residue in a small amount of the above-exemplified inert solvent, charging the solution onto a column of Sephadex LH-20 (dextran gel, a product of Pharmacia Fine Chemicals AB) or Bio-Rad S-series (styrene-divinylbenzene polymer, a product of Bio-Rad Company) and developing the column with the same solvent, or as required, further charging the eluates onto a silica gel column. In this manner, the compound of formula (III) can be isolated from the reaction mixture.

Specific examples of the compound of formula (III) produced by the process of this invention include the following in addition to the compounds specifically illustrated in Examples to be given hereinafter.

Sodium 3-(2-acetoxyethylthio)-6-ethyl-7-oxo-1-azabicyclo[3.2.0.]hept-2-ene-2-carboxylate,

sodium 3-(2-acetoxyethylthio)-6-isopropyl-7-oxo-1-azabicyclo[3.2.0]hept-2-ene-2-carboxylate,

sodium 3-(2-ethoxyethylthio)-6-ethyl-7-oxo-1-azabicyclo[3.2.0]hept-2-ene-2-carboxylate,

sodium 3-(2-ethoxyethylthio)-6-(1-methyl-1-hydroxy)ethyl-7-oxo-1-azabicyclo[3.2.0]hept-2-ene-2-carboxylate,

sodium 3-(2-phenoxyethylthio)-6-ethyl-7-oxo-1-azabicyclo[3.2.0]hept-2-ene-2-carboxylate,

pivaloyloxymethyl 3-(2-N,N-dimethylaminoethylthio)-6-(1-acetoxymethylisopropyl)-7-oxo-1-azabicyclo-[3.2.0]hept-2-ene-2-carboxylate,

sodium 3-(2-N,N-dimethylaminoethylthio)-6-(1-hydroxy-1-methyl)ethyl-7-oxo-1-azabicyclo[3.2.0]hept-2-ene-2-carboxylate,

phthalidyl 3-(2-phenylacetamidoethylthio)-6-(1-acetoxymethylisopropyl)-7-oxo-1-azabicyclo[3.2.0]hept-2-ene-2-carboxylate,

sodium 3-(2-phenylacetamidoethylthio)-6-isopropyl-7-oxo-1-azabicyclo[3.2.0]hept-2-ene-2-carboxylate,

3-furfurylthio-6-(1-acetoxy)ethyl-7-oxo-1-azabicyclo[3.2.0]hept-2-ene-2-carboxylic acid,

3-(3-hydroxypyridin-2-yl)methylthio-6-(1-methoxymethyloxy)ethyl-7-oxo-1-azabicyclo[3.2.0]hept-2-ene-2-carboxylic acid,

3-(5-aminopyridin-2-yl)thio-6-(1-methoxycarbonyloxy)ethyl-7-oxo-1-azabicyclo[3.2.0]hept-2-ene-2-carboxylic acid,

phthalidyl 6-(1-tetrahydropyranyloxy)ethyl-3-(1-oxy-4-nitropyridin-3-yl)thio-7-oxo-1-azabicyclo[3.2.0]-hept-2-ene-2-carboxylate,

pivaloyloxymethyl 3-(imidazol-4-yl)methylthio-6-(1-hydroxy-1-methyl)ethyl-7-oxo-1-azabicyclo[3.2.0]-hept-2-ene-2-carboxylate,

3-(1-methyltetrazol-5-yl)thio-6-(1-methoxy)-ethyl-7-oxo-1-azabicyclo[3.2.0]hept-2-ene-2-carboxylic-acid,

3-(1-methyltetrazol-5-yl)methylthio-6-(1-benzyloxy)ethyl-7-oxo-1-azabicyclo[3.2.0]hept-2-ene-2-carboxylic acid,

3-(4-aminopyrimidin-2-yl)-6-(1-propionyloxy)-ethyl-7-oxo-1-azabicyclo[3.2.0]hept-2-ene-2-carboxylic-acid,

pivaloyloxymethyl 3-(2-imidazol-1-yl)ethylthio-6-(1-acetoxy)ethyl-7-oxo-1-azabicyclo[3.2.0]hept-2-ene-2-carboxylate,

3-(2-imidazol-1-yl)ethylthio-6-ethyl-7-oxo-1-azabicyclo[3.2.0]hept-2-ene-2-carboxylic acid,

6-(1-hydroxy-1-methyl)ethyl-3-(5-aminothiazol-2-yl)thio-7-oxo-1-azabicyclo[3.2.0]hept-2-ene-2-carboxylic acid,

methyl 6-(1-benzyloxy)ethyl-3-(5-nitropyrimidin-2-yl)thio-7-oxo-1-azabicyclo[3.2.0]hept-2-ene-2-carboxylate,

pivaloyloxymethyl 6-(1-methoxycarbonyloxy)ethyl-3-(5-nitropyrimidin-2-yl)methylthio-7-oxo-1-azabicyclo-[3.2.0]-hept-2-ene-2-carboxylate,

pivaloyloxymethyl 6-(1-tetrahydrofuranyloxy)-ethyl-3-(1-methyl-4-nitrotriazol-5-yl)thio-7-oxo-1-azabicyclo-[3.2.0]hept-2-ene-2-carboxylate,

phthalidyl 6-(1-propionyloxy)ethyl-3-(1-methyl-4-nitrotriazol-5-yl)methylthio-7-oxo-1-azabicyclo-[3.2.0]-hept-2-ene-2-carboxylate,

6-ethyl-3-(4-amino-2-methyl-triazol-5-yl)thio-7-oxo-1-azabicyclo[3.2.0]hept-2-ene-2-carboxylic acid,

6-ethyl-3-(4-amino-2-methyl-triazol-5-yl)methylthio-7-oxo-1-azabicyclo[3.2.0]hept-2-ene-2-carboxylic acid,

3-(2-imidazolylthio)-6-(1-hydroxy-1-methylethyl)-7-oxo-1-azabicyclo[3.2.0]hept-2-ene-2-carboxylic acid,

phthalidyl 3-(4-imidazolylthio)-6-(2-acetoxy-isopropylidene)-7-oxo-1-azabicyclo[3.2.0]hept-2-ene-2-carboxylate,

3-(2-pyrrolylthio)-6-isopropyl-7-oxo-1-azabicyclo[3.2.0]hept-2-ene-2-carboxylic acid,

3-(3-pyrrolylthio)-6-(1-butoxyethyl)-7-oxo-1-azabicyclo[3,2,0]hept-2-ene-2-carboxylic acid,

3-(4-pyridylthio)-6-isopropyl-7-oxo-1-azabicyclo-[3.2.0]hept-2-ene-2-carboxylic acid,

3-(2-pyridylthio)-6-(1-hydroxy-1-methylethyl)-7-oxo-1-azabicyclo[3.2.0]hept-2-ene-2-carboxylic acid,

pivaloyloxymethyl 3-(2-pyridylthio)-6-(2-acetoxy-isopropylidene)-7-oxo-1-azabicyclo[3.2.0]hept-2-ene-2-carboxylate,

p-nitrobenzyl 3-(2-pyridylthio)-6-isopropyl-7-oxo-1-azabicyclo[3.2.0]hept-2-ene-2-carboxylate,

phthalidyl 3-(2-pyrimidinylthio)-6-(1-methoxy-methyl-oxyethyl)-7-oxo-1-azabicyclo[3.2.0]hept-2-ene-2-carboxylate,

pivaloyloxymethyl 5,6-cis-3-(2-pyrimidinylthio)-6-(1-methoxyethyl)-7-oxo-1-azabicyclo[3.2.0]hept-2-ene-2-carboxylate,

3-(2-pyrimidinylthio)-6-isopropyl-7-oxo-1-azabicyclo[3.2.0]hept-2-ene-2-carboxylic acid,

3-(2-pyrimidinylthio)-6-(1-acetoxyethyl)-7-oxo-1-azabicyclo[3.2.0]hept-2-ene-2-carboxylic acid,

3-(2-pyrimidinylthio)-6-(1-propionyloxyethyl)-7-oxo-1-azabicyclo[3.2.0]hept-2-ene-2-carboxylic acid,

3-(2-pyrimidinylthio)-6-(1-butoxyethyl)-7-oxo-1-azabicyclo[3.2.0]hept-2-ene-2-carboxylic acid,

sodium 3-phenylthio-6-(1-tetrahydropyranyloxyethyl)-7-oxo-1-azabicyclo[3.2.0]hept-2-ene-2-carboxylate,

sodium 3-phenylthio-6-(1-hydroxy-1-methylethyl)-7-oxo-1-azabicyclo[3.2.0]hept-2-ene-2-carboxylate,

6-ethylidene-3-(4-pyridyl)thio-7-oxo-1-azabicyclo-[3.2.0]hept-2-ene-2-carboxylic acid, and

6-ethylidene-3-(imidazol-4-yl)methylthio-7-oxo-1-azabicyclo[3.2.0]hept-2-ene-2-carboxylic acid.

Of the compounds of formula (I) which are used as starting materials in the process of this invention, those in which $R_1$ and $R_3$ are hydrogen atoms, $R_2$ is a hydroxyl group which may be protected, $R_4$ is $-CH_2-CH_2-$, and Y is a protected amino group, namely compounds of the following formula

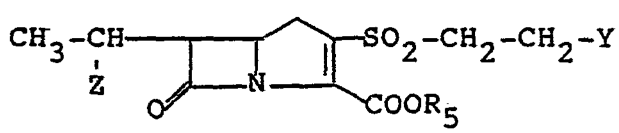

(I-3)

wherein Z represents a hydroxyl group which may be protected, and $R_5$ and Y are as defined above,

are known compounds which are generally disclosed in the specification of U.S. Patent No. 4,372,965.

But this specification fails to describe anything about compounds of formula (I-3) in which Y at least represents a group of the formula

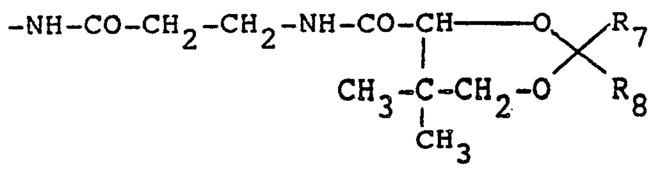

wherein $R_7$ and $R_8$ are as defined hereinabove,

and therefore, such compounds are novel compounds.

Compounds of formula (I) in which $R_1$ through $R_4$ are defined hereinabove excepting the definitions given immediately above are novel compounds not described in the prior literature. These novel compounds can be produced by the same method as in the case of producing the above known compounds. Specifically, the compounds of formula (I) can be produced by S-oxidation of compounds of the following general formula (VI)

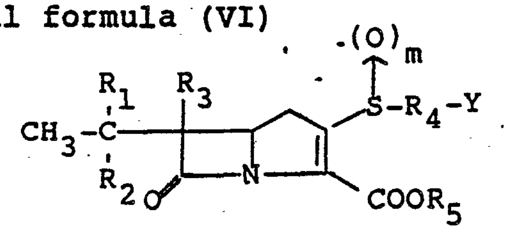

(VI)

wherein m is 0 or 1, and $R_1$, $R_2$, $R_3$, $R_4$, $R_5$ and Y are as defined hereinabove.

S-oxidation of the compounds of formula (VI) can be carried out by methods known <u>per se</u>, for example by methods frequently utilized in the S-oxidation of sulfur-containing beta-lactam type antibiotics such as penicillins and cephalosporins.

For example, the compound of formula (VI) is reacted with a mild oxidizing agent which does not sub stantially act on the 7-oxo-1-azabicyclo[3.2.0]-hept-2-ene-2-carboxylic acid skeleton, such as

benzoic acid, phenyldichloroiodide, hydrogen peroxide, selenium dioxide and sodium meta-periodide. Organic peracids are suitable as such mild oxidizing agents, especially suitable being perbenzoic acid and m-chloroperbenzoic acid. Substituted perbenzoic acids such as m-chloroperbenzoic acid are especially preferred.

The reaction of the compound of formula (VI) with the oxidizing agent is advantageously carried out in an inert solvent such as methylene chloride, chloroform or carbon tetrachloride at a mild temperature, for example, 0 to 50°C, preferably 0 to 25°C. Under these conditions, the reaction can be terminated usually in 3 minutes to 3 hours.

The amount of the oxidizing agent used to oxidize the compound of formula (VI) can be varied widely depending upon the type of the oxidizing agent, the reaction conditions, etc. Generally, it is 2 to 5 mole equivalents, preferably 2 to 3 mole equivalents, per mole of the compound of formula (VI) when m is 0, and 1.0 to 1.8 mole equivalents, preferably 1.0 to 1.3 mole equivalents, per mole of the compound of formula (VI) when m is 1.

After the reaction, the compound of formula (I) as an S-oxidation product can be isolated and purified by various methods known per se. Usually, it can be isolated from the reaction mixture by methods which are frequently utilized in the isolation and purification of antibiotics. These compounds of formula (I) can be used in the aforesaid reaction.

When in the S-oxidation of the compound of formula (I) or its salt, $R_2$ represents a hydroxyl group which may be protected or a hydroxymethyl group which may be protected, and a primary or secondary hydroxyl group exists as a substituent on the 6-

position side chain (for example, 2-hydroxyisopropylidene or 1-hydroxyethyl), the hydroxyl group is preferably protected. Furthermore, when the protected amino group for Y has basicity (for example, when Y represents an amino group which is mono- or disubstituted by alkyl or aralkyl), the S-oxidation may simultaneously oxidize the N-atom of the amino group, thus forming an N-oxide. The reaction in accordance with this invention similarly proceeds even when the protected amino group Y is oxidized.

Specific examples of the compounds of formula (I) so produced are given below in addition to those given in Examples below.

Benzyl 3-(2-acetamino)ethylthio-6-ethyl-7-oxo-1-azabicyclo[3.2.0]hept-2-ene-2-carboxylate sulfone,

phthalidyl 3-(2-acetamino)ethylthio-6-ethyl-7-oxo-1-azabicyclo[3.2.0]hept-2-ene-2-carboxylate sulfone,

methyl 3-(2-acetamino)ethylthio-6-ethyl-7-oxo-1-azabicyclo[3.2.0]hept-2-ene-2-carboxylate sulfone,

benzyl 3-(2-acetamino)ethenylthio-6-ethyl-7-oxo-1-azabicyclo[3.2.0]hept-2-ene-2-carboxylate sulfone,

phthalidyl 3-(2-acetamino)ethenylthio-6-ethyl-7-oxo-1-azabicyclo[3.2.0]hept-2-ene-2-carboxylate sulfone,

benzyl 6-ethyl-3-pantetheinyl-7-oxo-1-azabicyclo[3.2.0]hept-2-ene-2-carboxylate sulfone,

p-nitrobenzyl 3-diacetylpantetheinyl-6-ethyl-7-oxo-1-azabicyclo[3.2.0]hept-2-ene-2-carboxylate sulfone,

phthalidyl 6-ethyl-3-isopropylidenepantetheinyl-7-oxo-1-azabicyclo[3.2.0]hept-2-ene-2-carboxylate sulfone,

pivaloyloxymethyl 3-(2-acetamino)ethylthio-6-(1-acetoxyethyl)-7-oxo-1-azabicyclo[3.2.0]hept-2-ene-2-carboxylate sulfone,

p-nitrobenzyl 6-(1-acetoxyethyl)-3-(2-propionylamino)ethylthio-7-oxo-1-azabicyclo[3.2.0]hept-2-ene-2-carboxylate sulfone,

phthalidyl 6-(1-tetrahydropyranyloxyethyl)-3-(2-propionylamino)ethenylthio-7-oxo-1-azabicyclo[3.2.0]hept-2-ene-2-carboxylate sulfone,

methyl 6-(1-methoxyethyl)-3-(isopropylidenepantetheinyl)-7-oxo-1-azabicyclo[3.2.0]hept-2-ene-2-carboxylate sulfone,

phthalidyl 6-(1-acetoxyethyl)-3-(isopropylidenepantetheinyl)-7-oxo-1-azabicyclo[3.2.0]hept-2-ene-2-carboxylate sulfone,

phthalidyl 3-(2-acetamino)ethylthio-6-(1-hydroxy-1-methyl)ethyl-7-oxo-1-azabicyclo[3.2.0]hept-2-ene-2-carboxylate sulfone,

phthalidyl 3-(2-acetamino)ethenylthio-6-(1-hydroxy-1-methyl)ethyl-7-oxo-1-azabicyclo[3.2.0]hept-2-ene-2-carboxylate sulfone,

pivaloyloxymethyl 3-(2-acetamino)ethylthio-6-(2-acetoxyisopropylidene)-7-oxo-1-azabicyclo[3.2.0]-hept-2-ene-2-carboxylate sulfone,

phthalidyl 3-(2-acetamino)ethenylthio-6-(2-methoxycarbonyloxyisopropylidene)-7-oxo-1-azabicyclo-[3.2.0]hept-2-ene-2-carboxylate sulfone,

pivaloyloxymethyl 3-(2-acetamino)ethylthio-6-isopropylidene-7-oxo-1-azabicyclo[3.2.0]hept-2-ene-2-carboxylate sulfone,

phthalidyl 3-(diacetylpantetheinyl)-6-ethylidene-7-oxo-1-azabicyclo[3.2.0]hept-2-ene-2-carboxylate sulfone,

p-nitrobenzyl 6-ethylidene-3-(ditosylpantetheinyl)-7-oxo-1-azabicyclo[3.2.0]hept-2-ene-2-carboxylate sulfone, and

p-nitrobenzyl 6-ethylidene-3-(dimesylpantetheinyl)-7-oxo-1-azabicyclo[3.2.0]hept-2-ene-2-carboxylate sulfone.

Some of the compounds of formula (VI) used in the S-oxidation are known (see U. S. Patent Nos. 4,318,916, 4,168,268, 4,162,323, 4,368,203, 4,269,873, 4,264,736, 4,264,734, 4,264,735, 4,387,052, 4,341,706, 4,234,920, 4,123,547 and 4,150,145 and European Laid-Open Patent Publications Nos. 1,627, 1,628 and 44,142), or disclosed in the Journal of Antibiotics, 34, pages 909 et seq. (1981). Novel compounds of formula (VI) can be produced in the same way as in the production of the known compounds.

In particular, among the novel compounds mentioned above, those of the following formula

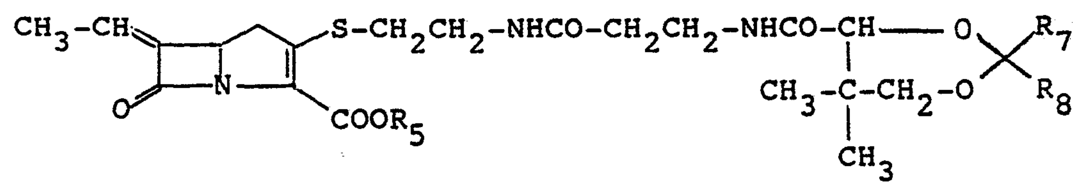

wherein $R_5$, $R_6$ and $R_7$ are as defined above, can be prepared by esterifying antibiotic OA-6129 $B_1$ or $B_2$ represented by the following formula

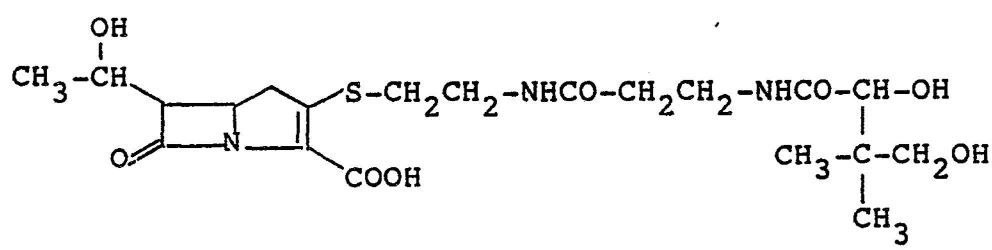

produced by the method described in European Laid-Open Patent Publication No. 44,142, at its carboxyl group by known methods for production of carboxylic acid esters, reacting the resulting ester

having the group $R_5$ with a ketone or aldehyde derivative to etherify the hydroxyl groups at the alpha- and gamma-positions of the panthoyl group simultaneously and thus obtain a cyclic ketal or cyclic acetal, and further subjecting the resulting compound to dehydration reaction in an inert solvent using a combination of triphenylphosphine and an azodicarboxylic acid diester (see, for example, Japanese Laid-Open Patent Publication No. 32478/1981).

Although in the present specification and claims, all chemical formulae are described in their planar structure for the sake of simplicity, it should be understood that the compounds of formula (I) as the starting materials and the compounds of formula (III) as the products include stereoisomers and optical isomers. Thus, for example, the above formula (III) includes the following two stereoisomers and a mixture of these.

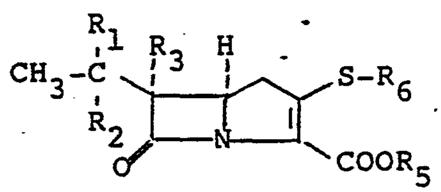

5,6-cis isomer

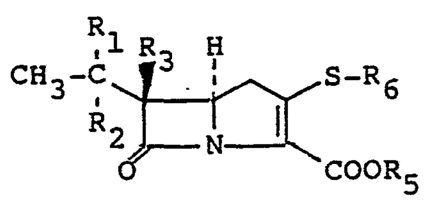

5,6-trans isomer

When $R_1$ represents a hydroxyl group, the carbon atom to which $R_1$ is bonded is an asymmetric carbon atom. In this case, the compounds of formula (III) may take both S- and R- steric configurations.

The compounds of formula (III) or the salts

thereof obtained by the process of this invention have excellent antibacterial activity, and can be used as an active ingredient of antibacterial agents for the prevention, therapy and/or treatment of infections caused by Gram-positive and Gram-negative bacteria not only in man but also in other animals such as mammals, poultry and fish.

The following Examples illustrate the present invention specifically.

Example 1

(1) Synthesis of PS-5·p-nitrobenzyl ester· sulfone (via the sulfoxide):-

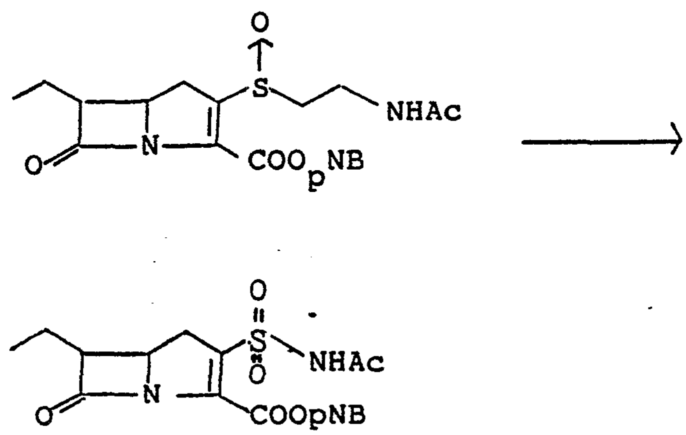

520 mg of PS-5·p-nitrobenzyl ester (to be abbreviated as "p-NB ester") sulfoxide compound was dissolved in 50 ml of methylene chloride, and with ice cooling and stirring, 20 ml of a methylene chloride solution containing 320 mg of m-chloroperbenzoic acid was added dropwise. The reaction mixture was warmed to room temperature and reacted for 1 hour.

The reaction mixture was again cooled with ice, and a methylene chloride solution containing 0.2 ml of triethylamine was slowly added dropwise. The temperature was returned to room temperature, and the mixture was stirred for 10 minutes.

The reaction mixture was diluted with 150 ml of methylene chloride, and washed with two 100 ml portions of a saturated aqueous solution of sodium bicarbonate, and further with 100 ml of a saturated aqueous solution of sodium chloride. The methylene chloride layer was dried over anhydrous sodium sulfate, and concentrated under reduced pressure.

The residue (10 g) was purified on a column of 10 g of silica gel to obtain 284 mg of the title compound having an UV absorption at Rf=0.58 in thin-layer chromatography (developing solvent: benzene/acetone=1/1).

(2) Synthesis of PS-5·pNB·sulfone:-

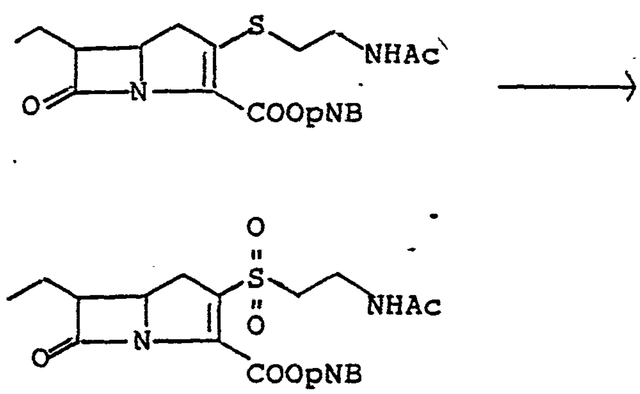

49 mg of PS-5 pNB ester was dissolved in 5 ml of methylene chloride, and with ice cooling and stirring, 2 ml of a methylene chloride solution containing 16 g of m-chloroperbenzoic acid was added dropwise. The temperature of the reaction mixture was returned to room temperature, and it was reacted for 1 hour.

The reaction mixture was again cooled with ice, and a methylene chloride solution containing 0.01 ml of triethylamine was slowly added dropwise. The temperature was returned to room temperature, and the mixture was stirred for 10 minutes.

The reaction mixture was diluted with 15 ml of methylene chloride, washed with two 10 ml portions of a saturated aqueous solution of sodium bicarbonate, and then with 10 ml of a saturated aqueous solution of sodium chloride. The methylene chloride layer was dried over anhydrous sodium sulfate, and concentrated under reduced pressure.

The residue was purified over a silica gel column (1.5 g) to give 27 mg of the title compound having a UV absorption at Rf=0.58 ion thin-layer chromatography (developing solvent: benzene/acetone =1/1).

The physicochemical properties of this compound are shown below.

NMR ($CDCl_3$, TMS) δ ppm:

1.10 (3H, t, J=7.5Hz, $C\underline{H_3}-CH_2-$),
1.85 (2H, m, $CH_3-C\underline{H_2}-$),
1.96 (3H, s, $NHCOC\underline{H_3}$),
3.15-3.85 (7H, m, $\underline{C-4H_2}$, C-6H, $S-C\underline{H_2}-C\underline{H_2}-N$),
4.15 (1H, dt, J=3.0Hz, J-9.0Hz, C-$\underline{5H}$),
5.42 (2H, s, $C\underline{H_2}-Ar$),
6.37 (1H, br, $\underline{NH}$),
7.60 (2H, d, J=8.0Hz, Ar$\underline{H}$),
8.21 (2H,d, J=8.0Hz, Ar$\underline{H}$).

UV ($CHCl_3$) $\lambda_{max}$: 269.5 nm (ε =11500).

Specific rotation $[\alpha]_D^{24}$: $-49.1^\circ$ (c=1.0, $CHCl_3$).

IR ($CHCl_3$) $\lambda_{max}$ $cm^{-1}$: 1800 (β-lactam), 1740 (ester), 1675 (amide), 1320 (sulfone).

Example 2

Synthesis of PS-5·pivaloyloxymethyl ester·sulfone:-

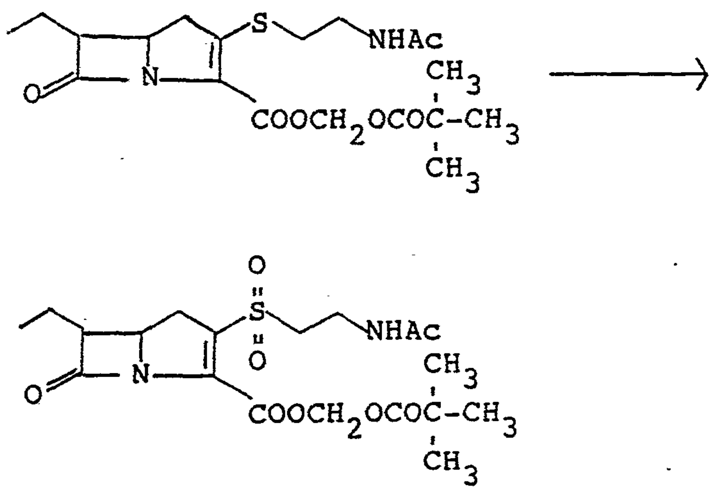

100 mg of PS-5·pivaloyloxymethyl ester was dissolved in 10 ml of methylene chloride, and with ice cooling and stirring, 4 ml of methylene chloride solutin containing 120 mg of m-chloroperbenzoic acid was added dropwise. The reaction mixture was warmed to room temperature and reacted for 1 hour.

The reaction mixture was again cooled with ice, and a methylene chloride solution containing 0.1 ml of triethylamine was slowly added dropwise. The temperature was returned to room temperature, and the mixture was stirred for 10 minutes.

The reaction mixture was diluted with 30 ml of methylene chloride, and washed with two 20 ml portions of a saturated aqueous solution of sodium bicarbonate, and further with 20 ml of a saturated aqueous solution of sodium chloride. The methylene chloride layer was dried over anhydrous sodium sulfate, and concentrated under reduced pressure.

The residue was purified on a silica gel column (2 g) to give 57.2 mg of the title compound having a UV absorpotion at Rf=0.52 in thin-layer chromatography (developing solvent: benzene/acetone=

1/1).

The physicochemical properties of the resulting compound are shown below.

NMR ($CDCl_3$, TMS), $\delta$ ppm:

1.00 (3H, t, J=7.5Hz, $CH_3$-$CH_2$-),
1.22 (9H, s, $C(CH_3)_3$),
1.80 (2H, m, $CH_3$-$CH_2$-),
1.97 (3H, s, $NHCOCH_3$),
3.10-3.90 (7H, m, C-4$H_2$, C-6H, S-$CH_2$-$CH_2$-N),
4.09 (1H, dt, J=3.0Hz, J=9Hz, C-5H),
5.88 (2H, s, O-$CH_2$-O),
6.82 (1H, br, NH).

UV ($CHCl_3$) $\lambda_{max}$: 283 nm ($\varepsilon$ =3300).

IR ($CHCH_3$) $\nu_{max}$ $cm^{-1}$: 1790 ($\beta$-lactam), 1750 (ester), 1670 (amide), 1325 (sulfone).

Mass (FD): 445 (M+1).

Example 3

Synthesis of dehydro·PS-5·pivaloyloxymethyl·sulfoxide:-

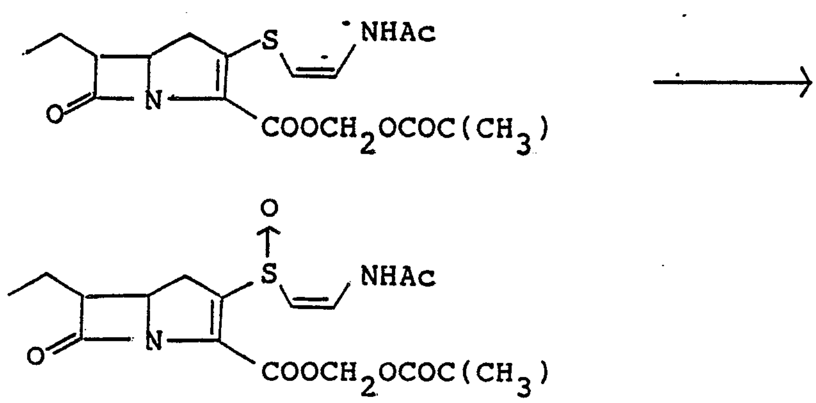

17.7 mg of dehydro·PS-5·pivaloyloxymethyl ester compound was dissolved in 3 ml of anhydrous methylene chloride, and the solution was cooled to -50°C. In an atmosphere of nitrogen, 1 ml of an

anhydrous methylene chloride solution containing 1 mg of m-chloroperbenzoic acid was slowly added dropwise, and the reaction was carried out at the above temperature for 20 minutes.

The reaction mixture was poured into methylene chloride, and washed three times with a saturated aqueous solution of sodium bicarbonate, and further washed with s saturated aqueous solution of sodium chloride. The organic layer was dried over anhydrous sodium sulfate, and concentrated under reduced pressure.

The residue was subjected to silica gel chromatography to give 9.5 mg of the title compound.

The physicochemical properties of this compound are shown below.

NMR ($CDCl_3$, TMS), δ ppm:

1.02 (3H, t, J=7.5Hz, $C\underline{H_3}-CH_2-$),
1.21 (9H, s, $C(C\underline{H_3})_3$,
1.81 (2H, m, $CH_3-C\underline{H_2}-$),
2.08 (3H, s, $NHCOC\underline{H_3}$),.
2.85-3.80 (3H, m, C-4$H_2$, C-6H),
4.00 (1H, m, C-5H),
5.21 (1H, d, J=10.0Hz, S-C$\underline{H}$=CH),
5.78, 5.85, 5.92 (total 2H, m, $COOCH_2O-$),
7.43 (1H, dd, J=12.0Hz, J=10.0Hz, S-CH=CH),
10.00 (1H, br, NH).

Example 4

Synthesis of dehydro·PS-5·puvaloyloxymethyl sulfone:-

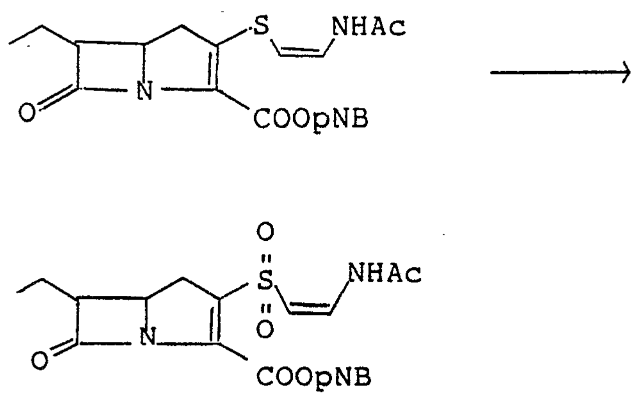

26 mg of dehydro·PS-5·pivaloyloxymethyl ester was dissolved in 5 ml of methylene chloride, and with ice cooling and stirring, 1.5 ml of a methylene chloride solution containing 32 mg of m-chloroperbenzoic acid was added dropwise. The reaction mixture was warmed to room temperature, and reacted for 1 hour.

The reaction mixture was again cooled with ice, and a methylene chloride solution containing 0.03 ml of triethylamine was slowly added dropwise. The temperature was returned to room temperature, and the mixture was stirred for 10 minutes.

. The reaction mixture was diluted with 15 ml of methylene chloride and washed twice with 10 ml of a saturated aqueous solution of sodium bicarbonate and then with 10 ml of a saturated aqueous solution of sodium chloride. The methylene chloride layer was dried over anhydrous sodium sulfate, and concentrated under reduced presssure.

The residue was purified on a silica gel column (1.5 g) to give 12.3 mg of the title compound having a UV absorption at Rf=0.63 in thin-layer chromatography (developing solvent: benzene/acetone=3/1).

The physicochemical properties of the result-

ing compound are shown below.

NMR ($CDCl_3$, TMS), δ (ppm):

1.03 (3H, t, J=7.5Hz, $CH_3-CH_2-$),
1.80 (2H, m, $CH_3-CH_2-$),
2.17 (3H, s, $NHCOCH_3$),
3.05-3.70 (H, m, C-4$H_2$, C-6H),
4.13 (1H, dt, J=3.0Hz, J=9.0Hz, C-5H),
5.37 (2H, s, $CH_2$-Ar),
5.48 (1H, d, J=10.0Hz, S-CH=CH),
7.55 (1H, dd, J=10.0Hz, J=12.0Hz, S-CH=CH),
7.60 (2H, d, J=8.0Hz, ArH),
8.21 (2H, d, J=8.0Hz, ArH),
9.63 (1H, br, J=12.0Hz, NH).

UV ($CHCl_3$) $\lambda_{max}$: 260 nm (ε =18000).

IR ($CHCl_3$) $\nu_{max}$ $cm^{-1}$: 1790 (β-lactam), 1725 (ester, amide), 1310 (sulfone).

Example 5

Production of pNB ester of antibiotic OA-6129A (the deoxy product at the 8-position hydroxyl group of antibiotic OA-6129$B_1$ or $B_2$; see, for example, European Laid-Open Patent Publication No 48,999):-

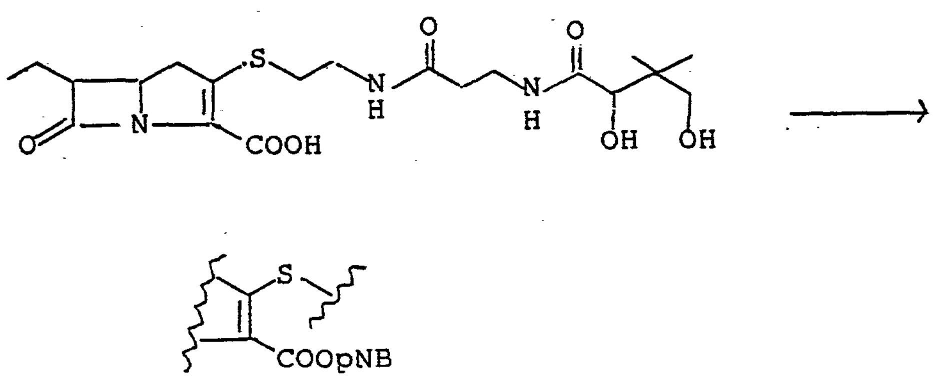

63.5 mg of antibiotic OA-6129A was dissolved in 9.0 ml of dimethylformamide, and with ice cooling, 0.2 ml of triethylamine was added. With stirring, 1.5 ml of a dimethylformamide solution containing 285 mg of p-nitrobenzyl bromide was added. The reaction was carried out at the same temperature for 30 minutes, and then at room temperature for 3 hours.

The reaction mixture was poured into 100 ml of ethyl acetate and washed with 20 ml of 0.01M phosphate buffer (pH 8.4) saturated with sodium chloride. The aqueous layer was again extracted with 100 ml of methylene chloride. The extracts were combined, dehydrated over anhydrous sodium sulfate, and then distilled under reduced pressure to remove the solvents.

The residue was dissolved in a small amount of methylene chloride, and the solution was adsorbed onto a column of 12 g of silica gel filled with benzene/acetone (=1/1), and successively developed with a 1:1 mixture of benzene and acetone, a 1:3 mixture of benzene and acetone, and then with acetone. Those eluates which were obtained with acetone and showed a UV absorption at an Rf value of 0.33 in silica gel thin-layer chromatography developed with a 1:1 mixed solvent of benzene and acetone were collected and dried under reduced pressure to give 36.3 mg of the title compound.

The resulting pNB ester of antibiotic OA-6129A showed the following physicochemical properties.

(1) Spcific rotation

$[\alpha]_D^{24}$: 37.5° (c=1.0, $CH_2Cl_2$).

(2) UV absorption spectrum

$\lambda_{max}^{CH_2Cl_2}$ nm(ε): 319 (8400), 270 (10500).

(3) IR absorption spectrum

$\nu_{max}^{CH_2Cl_2}$ $cm^{-1}$: 1770 (β-lactam),
1700 (ester),

1665 (amide).

(4) NMR spectrum (solvent: $CD_2Cl_2$; internal standard: TMS), δ (ppm):

0.87 (3H, s, $-\underline{CH_3}-\overset{CH_3}{\overset{|}{C}}-$),
0.95 (3H, s, $CH_3-\overset{\underline{CH_3}}{\overset{|}{C}}-$),
1.04 (3H, t, J=7.5Hz, $CH_2-\underline{CH_3}$),
1.5-2.2 (3H, m, $\underline{CH_2}-CH_3$, OH),
2.40 (2H, t, J=6.5Hz, $N-CH_2-\underline{CH_2}-CO$),
2.8-3.7 (12H, m, C-4$H_2$, C-6H, $S-\underline{CH_2}-\underline{CH_2}-N$, $N-\underline{CH_2}-CH_2-CO$, $C-\underline{CH_2}-OH$, OH or NH),
3.94 (2H, m, C-5H, HO-C$\underline{H}$-CO),
4.17 (1H, br, NH or OH),
5.19 (1H, d, J=14Hz, $\underline{CH}$·H-Ar),
5.45 (1H, d, J=14Hz, CH·$\underline{H}$-Ar),
6.74 (1H, br, NH),
7.63 (2H, d, J=9Hz, ArH),
8.18 (2H, d, J=9Hz, ArH).

Example 6

Production of the isopropylidene derivative of the pNB ester of antibiotic OA-6129A:-

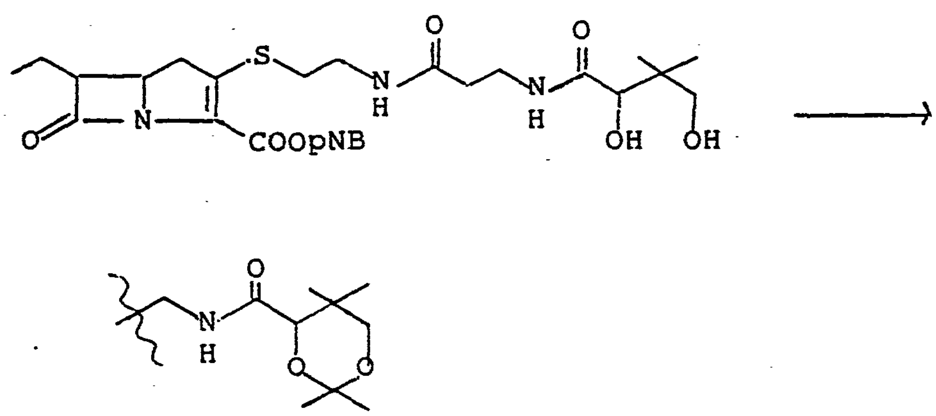

110 mg of the pNB ester of antibiotic OA-6129A

was dissolved in 10 ml of acetone, and 0.5 ml of 2,2-dimethoxypropane and 20 mg of anhydrous sodium sulfate were added. With stirring at room temperature, 40 mg of anhydrous p-toluenesulfonic acid was added, and the reaction was carried out at this temperature for 3 hours.

To the reaction mixture was added dropwise 0.1 ml of triethylamine. The mixture was stirred for 5 minutes, and then poured into 50 ml of ethyl acetate. It was then washed with 20 ml of 0.1M phosphate buffer (pH 8.4) and further with 20 ml of the same buffer (pH 6.8).

The extract was dehydrated over anhydrous sodium sulfate, and then distilled under reduced pressure to remove the solvent. The residue was dissolved in a small amount of methylene chloride, and the solution was adsorbed onto a column of 5 g of silica gel filled with benzene/acetone (5/1). The column was successively developed with benzene/acetone mixtures at ratios of 5:1, 3:1, 1:1, 2:1 and 1:5.

Eluates obtained with a 1:1 benzene-acetone mixture were collected and concentrated to give 72.0 mg of the titile compound which showed a UV absorption at an Rf value of 0.56 in silica gel thin-layer chromatography developed with a 1:1 mixed solvent of benzene and acetone.

The physicochemical properties of the resulting compound are shown below.

(1) Specific rotation

$[\alpha]_D^{24}$: $34.9^o$ (c=1.0, $CHCl_3$).

(2) UV absorption spectrum

$\lambda_{max}^{CHCl_3}$ nm(ε): 319 (6200), 270 (9800).

(3) NMR spectrum (solvent $CDCl_3$, internal standard: TMS), δ(ppm):

0.97(3H, s, $\underline{CH_3}-\overset{CH_3}{\overset{|}{C}}-$),

 

1.03 (3H, s, 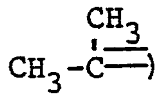),

1.07 (3H, t, J=7.5Hz, $CH_2$-$CH_3$),

1.40 (3H, s, 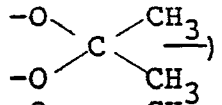),

1.43 (3H, s, 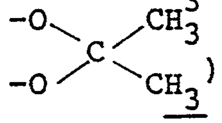),

1.7-2.0 (2H, m, -$CH_2$-$CH_3$),

2.43 (2H, t, J=6.5Hz, N-$CH_2$-$CH_2$-CO),

2.5-3.8 (11H, $C_4$-$H_2$, $C_5$-H, -S-$CH_2$-$CH_2$-N, N-$CH_2$-$CH_2$-CO, C-$CH_2$-O-),

3.97 (1H, dt, J=3Hz, 9Hz, $C_5$-H),

4.03 (1H, s, COCH-O-),

5.20 (1H, d, J=14Hz, CH·H-Ar),

5.49 (1H, d, J=14Hz, CH·H-Ar),

6.52 (1H, br, NH),

6.93 (1H, br, NH),

7.58 (2H, d, J=9Hz, ArH),

8.15 (2H, d, J=9Hz, ArH).

(4) IR absorption spectrum (solvent $CHCl_3$), $cm^{-1}$: 1770 (β-lactam), 1660 (amide).

Example 7

Production of the pNB ester of antibiotic OA-6129$B_2$:-

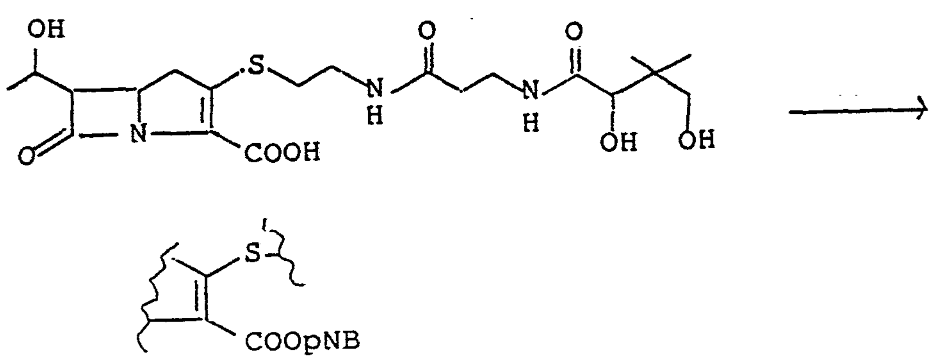

190 mg of the sodium salt of antibiotic OA-

6129$B_2$ was dissolved in 6.0 ml of dimethylformamide, and with ice cooling, 0.2 ml of triethylamine was added. With stirring, a dimethylformaide solution containing 210 mg of p-nitrobenzyl bromide was added. The reaction was carried out at the same temperature for 5 minutes and then at room temperature for 3 hours. The reaction mixture was poured into 100 ml of methylene chloride, and washed with two 20 ml portions of 0.1M phosphate buffer (pH 6.8) saturated with sodium chloride. The aqueous layer was further extracted with 100 ml of methylene chloride. The extracts were combined, dehydrated over anhydrous sodium sulfate, and then distilled under reduced pressure to remove the solvent.

The residue was dissolved in a small amount of methylene chloride, and the solution was adsorbed onto a column of 6 g of silica gel filled with benzene/acetone (1/1). The column was developed successively with benzene/acetone mixed solvents at mixing ratios of 1:1, 1:2, 1:3, and 1:5, and with acetone. There was obtained 85 mg of the title compound which was eluted with benzene/acetone (1:5) and acetone and showed a UV absorption at an Rf value of 0.15 in silica gel thin-layer chromatography developed with benzene/acetone (1:4).

The physicochemical properties of the resulting compound are shown below.

(1) Specific rotation

$[\alpha]_D^{24}$ : 41.4° (c=1.0, dioxane).

(2) IR spectrum

$\nu_{max}^{KBr}$ $cm^{-1}$: 1760 (β-lactam), 1695 (ester), 1640 (amide).

(3) UV spectrum

$\lambda_{max}^{CH_2Cl_2}$ nm(ε): 320 (10500), 171 (10500).

(4) NMR spectrum (pyridine-$d_5$), δ (ppm):

Signals at δ 1-5 ppm are described below.

1.30 (6H, s, $\underline{CH_3}$-C-$\underline{CH_3}$),
1.55 (3H, d, J=7.0Hz, $\underline{CH_3}$-CH),
2.70 (2H, t, J=6.5Hz, NH-$CH_2$-$\underline{CH_2}$-CO),
2.90-4.05 (11H, m, C-4$H_2$, C-6H,
S-$\underline{CH_2}$-$\underline{CH_2}$-NH, NH-$\underline{CH_2}$-$CH_2$-CO,
O-$\underline{CH_2}$-C-),
4.10-4.50 (2H, m, C-5H, C-8H),
4.52 (1H, s, HO-CH-CO).

(5) Mass spectrum (FD)

m/z: 523 [(M+1)-$CH_3$-CH(OH)-CH=C=O].

<u>Example 8</u>

Production of the isopropylidene derivatives of the pNB ester of antibiotic OA-6129$B_2$:-

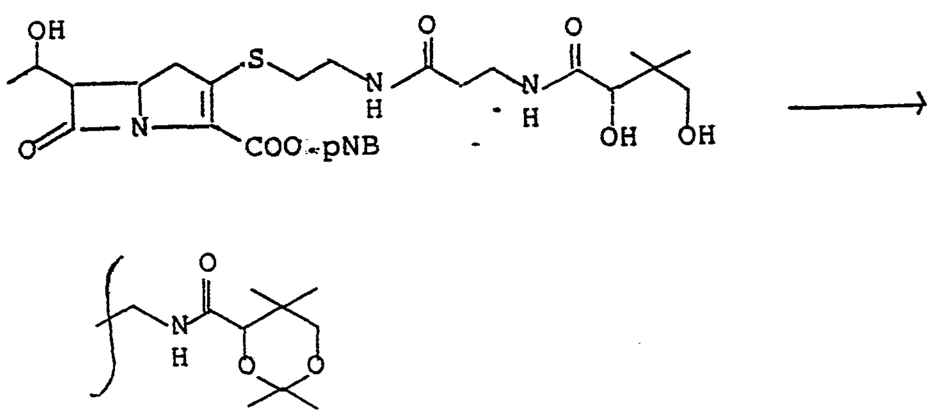

20 mg of the pNB ester of antibitic OA-6129$B_2$ was dissolved in a mixed solvent consisting of 5.0 ml of acetone, 2.0 ml of 2,2-dimethoxypropane

and 100 mg of anhydrous sodium sulfate. With stirring at room temperature, 0.5 mg of p-toluenesulfonic acid was added. The reaction was carried out for 30 minutes, and then 6 μl of triethylamine was added to the reaction mixture. The mixture was stirred for 5 minutes. The reaction mixture was distilled under reduced pressure, and 30 ml of methylene chloride was added to the residue. The mixture was washed with 20 ml of 0.1 phosphate buffer (pH 8.4). The extract was then dehyrated over anhydrous sodium sulfate, and distilled under reduced pressure. The residue was dissolved in a small amount of methylene chloride, and the solution was adsobed onto a column of 2 g of silica gel filled with benzene/acetone (2/1). The column was devloped successively with mixed solvents of benzene and acetone at mixing ratios of 2:1, 1:1 and 1:2. The eluates obtained with benzene/acetone mixed solvents at 1:1 and 1:2 were collected and concentrated to give 6.6 mg of the title compound which showed an Rf value of 0.64 in silica gel thin-layer chromatography developed with benzene/acetone (1:4).

The resulting compound had the following physicochemical properties.

(1) Specific rotation

$[\alpha]_D^{24}$: 55.1° (c=0.5, $CH_2Cl_2$)

(2) IR spectrum

$\nu_{max}^{CHCl_3}$ $cm^{-1}$: 1778 (β-lactam), 1700 (ester), 1668 (amide).

(3) UV spectrum

$\lambda_{max}^{CH_2Cl_2}$ nm(ε): 319 (9700), 270 (11900).

(4) NMR spectrum ($CDCl_3$), δ(ppm):

0.95 (3H, s, $\underline{CH_3}$-C($CH_3$)—),

1.02 (3H, s, $CH_3$-C($\underline{CH_3}$)—),

1.37 (3H, d, J=7.0Hz, $\underline{CH_3}$-CH),

O O

1.40 (3H, s, $CH_3$-C-$CH_3$),

O O

1.43 (3H, s, $CH_3$-C-$CH_3$),

2.41 (2H, t, J=6.5Hz, NH-$CH_2$-$CH_2$CO),

2.75-3.80 (11H, m, C-4Hz, C-6H, S-$CH_2$-$CH_2$NH, NH-$CH_2$-$CH_2$-CO, O-$CH_2$-C),

4.02 (1H, s, O-CH-CO),

4.00-4.30 (2H, m, C-5H, C-8H),

5.16 (1H, d, J=14.5Hz, CHH-Ar),

5.44 (1H, d, J=14.5Hz, CHH-Ar),

6.56 (1H, br, NH),

6.92 (1H, br, NH),

7.55 (2H, d, J=8.0Hz, Ar H),

8.12 (2H, d, J=8.0Hz, Ar H),

(5) Mass spectrum (FD)

OH

m/z: 563 [(M+1)-$CH_3$-CH-CH=C=O],

OH

562 (M-$CH_3$-CH-CH=C=O).

Example 9

Production of an S-oxide of the pNB ester of antibiotic OA-6129A:-

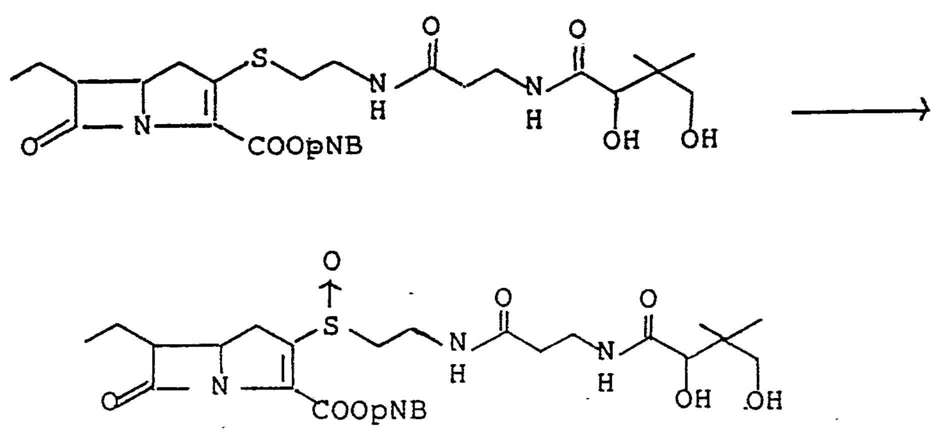

26 mg (0.044 mmole) of the pNB ester of OA-6129A was dissolved in 5 ml of methylene chloride. The solution was cooled to -35°C, and a methylene chloride solution containing 11 mg (0.066 mmole) of m-chloroperbenzoic acid was added dropwise. The reaction was carried out at the above temperature for 30 minutes. The reaction mixture was poured into 100 ml of methylene chloride, and washed with a saturated aqueous solution of sodium bicarbonate. The washing was adsorbed onto a column of 10 ml of silica gel filled with benzene/acetone (1/1). The column was eluted with benzene/acetone mixtures at ratios of 1:3 and 1:5. Those eluates which showed a UV absorption at an Rf value of 0.14 in silica gel thin-layer chromatography developed with acetone were concentrated under reduced pressure to give 16.8 mg of the title compound.

The resulting compound agreed in physicochemical properties with a compound obtained by subjecting to treatment with a peracid in the same way as above the pNB ester of (5R, 6R)-6-ethyl-3-(R)-pantetheinyl-7-oxo-1-aza-bicyclo[3.2.0]hept-2-ene-2-carboxylic acid which was obtained by reacting the S-oxide of the p-nitrobenzyl ester of PS-5 with D-pantetheine.

$[\alpha]_D^{24}$: 11.0° (c=1.0, $CH_2Cl_2$)

UV $\lambda_{max}^{CH_2Cl_2}$ nm(ε): 310sh (7500), 270 (12400).

IR $\nu_{max}^{CH_2Cl_2}$ $cm^{-1}$: 1790 (β=lactam), 1715 (ester), 1670 (amide).

NMR ($CDCl_2$)δ:

0.88 (3H, s, $\underline{CH_3}$-C(CH_3)—),

0105227

0.98 (3H, s, $CH_3$-C($CH_3$)—),

1.05 (3H, t, J=7.0Hz, $CH_2$-$CH_3$),

1.50-2.00 (3H, m, $CH_2$-$CH_3$, OH),

2.39 (2H, t, J=6.0Hz, CO-$CH_2$-$CH_2$-NH),

2.95-4.30 (14H, m, C-4$H_2$, C-5H, C-6H, S-$CH_2$-$CH_2$-NH, CO-$CH_2$-$CH_2$-NH, C-$CH_2$-O, O-CH-CO, OH),

5.10-5.60 (2H, m, $CH_2$-Ar),

6.70 (1H, m, NH),

7.30 (1H, m, NH),

7.62 (2H, m, Ar H),

8.20 (2H, m, Ar H),

MS (FD):

m/z 631 (M+Na), 609 (M+1).

Example 10

Production of O-acetylisopropylidene OA-6129$B_2$·pNB·sulfone:-

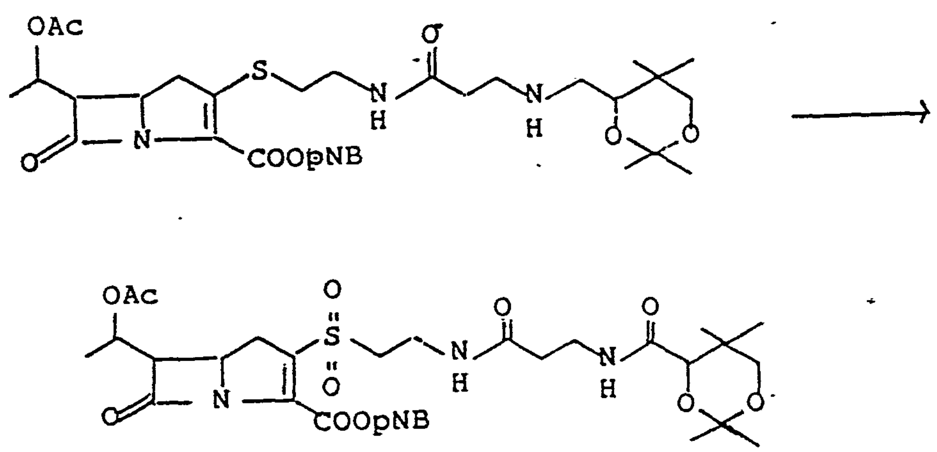

39 mg of O-acetylisopropylidene OA-6129$B_2$ pNB was dissolved in 8 ml of methylene chloride, and with ice cooling and stirring, a methylene chloride solution containing 29 mg of m-chloroperbenzoic acid was added dropwise. The reaction mixture was warmed to room temperature and reacted for 1 hour.

The reaction mixture was again cooled with

ice, and a methylene chloride solution containing 17 µl of triethylamine was added dropwise slowly. The temperature was returned to room temperature, and the mixture was stirred for 10 minutes.

The reaction mixture was diluted with 30 ml of methylene chloride, and washed with two 20 ml portions of a saturated aqueous solution of sodium bicarbonate and then with 20 ml of a saturated aqueous solution of sodium chloride. The methylene chloride layer was dried over anhydrous sodium sulfate, and concentrated under reduced pressure.

The residue was purified on a column of 2 g of silica gel to obtain 10 mg of the title compound which showed a UV absorption at an Rf value of 0.51 in thin-layer chromatography developed with benzene/-acetone (1/1).

The resulting compound had the following physicochemical properties.

NMR ($CDCl_3$, TMS), δ (ppm):

0.97 (3H, s, $\underline{CH_3}$-C-$CH_3$),
1.04 (3H, s, $CH_3$-C-$\underline{CH_3}$),
1.41 (3H, d, J=7.0Hz, CH-$\underline{CH_3}$),
1.40 (3H, s, $\underline{CH_3}$-C(O)(O)-$CH_3$),
1.43 (3H, s, $CH_3$-C(O)(O)-$\underline{CH_3}$),
2.43 (2H, t, J=6.5Hz, CO-$\underline{CH_2}$-$CH_2$-NH),
3.10-3.90 (11H, m, S-$\underline{CH_2}$-$\underline{CH_2}$-NH, CO-$CH_2$-$\underline{CH_2}$-NH-, C-4$H_2$, C-6H, C-$\underline{CH_2}$-O),
4.05 (1H, s, O-$\underline{CH}$-CO),
4.21 (1H, dt, J=3.0Hz, J=9.0Hz, C-5H),
5.22 (1H, dd, J=3.0Hz, J=4.5Hz, C-6H),
5.49 (2H, s, -$CH_2$-Ar),
6.68 (1H, br, NH),
6.90 (1H, br, NH),

7.68 (2H, d, J=9.0Hz, ArH),
8.18 (2H, d, J=9.0Hz, ArH).

IR ($CHCl_3$) $\nu_{max}$ $cm^{-1}$: 1800 (β-lactam), 1740 (ester), 1670 (amide), 1320 (sulfone).

Example 11

Production of anhydroisopropylidene·OA-6129$B_2$·pNB ester:-

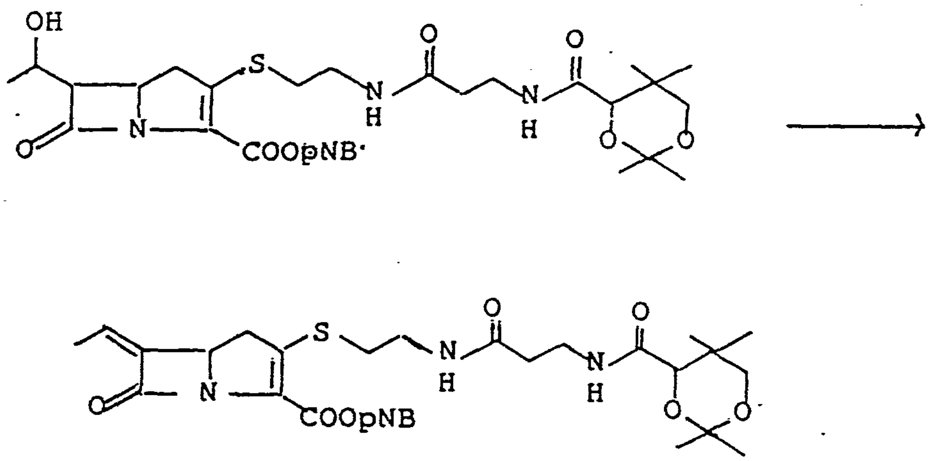

17 mg of OA-6129$B_2$ isopropylidene·pNB ester· was dissolved in 320 mg of triphenylphosphine and 3 ml of tetrahydrofuran. The solution was cooled with ice, and 7.5 µl of diethyl azodicarboxylate was added. The reaction was carried out at this temperature for 5 minutes and then at room temperature for 1 hour.

The reaction mixture was concentrated, dissolved in methylene chloride, washed with 0.01M phosphate buffer (pH 6.9), dried over anhydrous sodium sulfate, and concentrated under reduced pressure.

The residue was purified by silica gel column chromatography to give 12 mg of the title compound which showed an Rf value of 0.47 in thin-layer chromatography developed with benzene/acetone (1/1).

The resulting compound had the following

physicochemical properties.

NMR ($CDCl_3$, TMS), δ :

0.95 (3H, s, $CH_3$-C-$CH_3$),

1.02 (3H, s, $CH_3$-C-$CH_3$),

1.40 (3H, s, $CH_3$-C-$CH_3$),
O O

1.43 (3H, s, $CH_3$-C-$CH_3$),
O O

2.07 (3H, d, J=7.5Hz, $CH_3$-C=),

2.43 (2H, t, J=6.5Hz, $CH_2$-CO),

2.80-3.80 (10H, m, S-$CH_2$-$CH_2$-N, N-$CH_2$-$CH_2$-CO, $CH_2$-O, C-4$H_2$),

4.05 (1H, s, O-CH-CO),

4.70 (1H, t, J=9.0Hz, C-5H),

5.18 (1H, d, J=14.0Hz, CHH-Ar),

5.48 (1H, d, J=14.0Hz, CHH-Ar),

5.93 (1H, q, J=7.5Hz, $CH_3$-CH=),

6.50 (1H, br, NH),

7.95 (1H, br, NH),

7.62 (2H, J=8.5Hz, Ar),

8.17 (2H, J=8.5Hz, Ar).

UV ($CHCl_3$) $\nu_{max}$: 271.5 nm (ε=10300), 300 nm (ε=9600).

Specific rotation $[\alpha]_D^{23}$: $-15.4^{o}$ (c=1.0, $CHCl_3$).

IR ($CHCl_3$) $\nu_{max}$ $cm^{-1}$: 1760 (β-lactam), 1700 (ester), 1660 (amide).

Example 12

Production of the sulfoxide of anhydroisopropylidene·OA-6129$B_2$·pNB ester:-

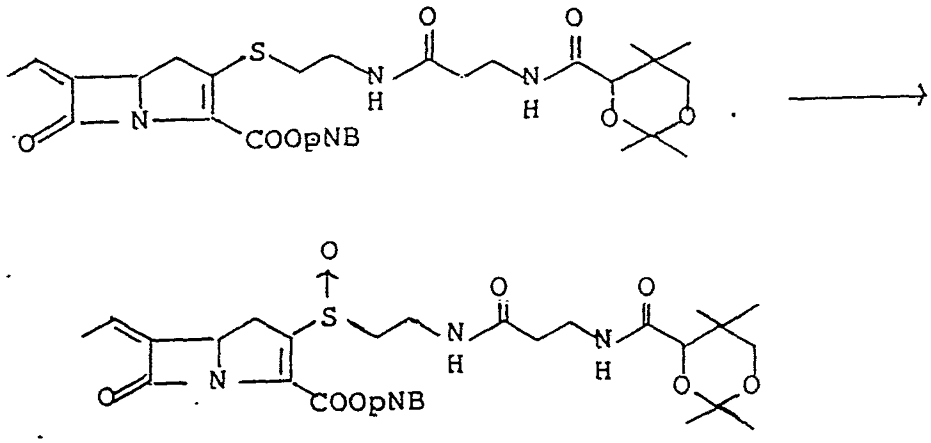

17 mg of anhydroisopropylidene·OA-6129$B_2$·pNB ester was dissolved in 3 ml of methylene chloride. The solution was cooled to -50°C, and 1 ml of a methylene chloride solution containing 9.3 mg of m-chloroperbenzoic acid was slowly added dropwise. The mixture was reacted at this temperature for 30 minutes.

To the reaction mixture was slowly added dropwise a methylene chloride solution containing 0.01 ml of triethylamine. The temperature was returned to room temperature, and the mixture was stirred for 10 minutes.

The reaction mixture was diluted with methylene chloride, and washed twice with a saturated aqueous solution of sodium bicarbonate and then with a saturated aqueous solution of sodium chloride. The methylene chloride layer was dried over anhydrous sodium sulfate, and concentrated under reduced pressure.

The residue was chromatographed over a column of 2 g of silica gel to give 9.8 mg of an isomeric mixture of the title compound which showed an Rf value of 0.31 and 0.26 in thin-layer chromatography developed

with benzene/acetone (1/2).

The resulting compound had the following physicochemical properties.

NMR ($CDCl_3$, TMS), δ (ppm):

0.97 (3H, s, $CH_3-C-CH_3$),
1.03 (3H, s, $CH_3-C-CH_3$),
1.41 (3H, s, $CH_3-C-CH_3$ / O O),
1.44 (3H, s, $CH_3-C-C-CH_3$ / O O),
2.13 (3H, d, J=7.5Hz, $CH_3-CH=C$),
2.45 (2H, t, J=6.5Hz, $N-CH_2-CH_2-CO$),
2.90-3.90 (10H, m, $C-4H_2$, $S-CH_2-CH_2-N$, $N-CH_2CH_2-CO$, $CH_2O$),
4.03 (1H, s, O-CH-CO),
4.80 (1H, m, C-5H),
5.23 (1H, d, J=14.5Hz, CHH-Ar),
5.50 (1H, d, J=14.5Hz, CHH-Ar),
6.11 (1H, m, $CH_3-CH=C$),
6.65 (1H, br, NH),
7.00 (1H, br, NH),
7.65 (2H, m, ArH),
8.20 (2H, m, ArH).

IR ($CHCl_3$) $\nu_{max}$ $cm^{-1}$:
1780 (β-lactam), 1715 (ester), 1670 (amide).

## Example 13

Production of the sulfone derivative of anhydro-isopropylidene·OA-6129$B_2$·pNB ester:-

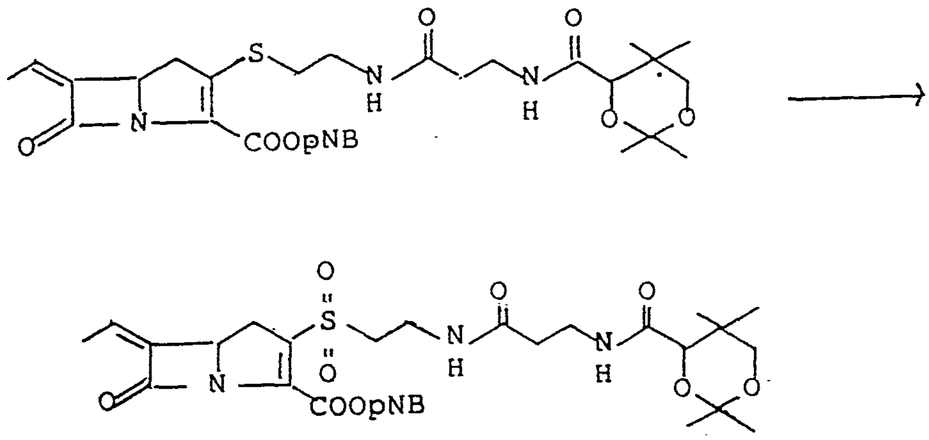

52 mg of anhydroisopropylidene·OA-6129$B_2$·pNB was dissolved in 5 ml of methylene chloride. With ice cooling and stirring, 15 mg of sodium bicarbonate powder was added, and 2 ml of a methylene chloride solution containing 46 mg of m-chloroperbenzoic acid was added dropwise. The reaction mixture was warmed to room temperature, and reacted for 1 hour.

The reaction mixture was again cooled with ice, and a methylene chloride solution containing 0.02 ml of triethylamine was slowly added dropwise. The temperature was returned to room temperature, and the mixture was stirred for 10 minutes.

The reaction mixture was diluted with 15 ml of methylene chloride, and washed with two 10 ml portions of a saturated aqueous solution of sodium bicarbonate and further with 10 ml of a saturated aqueous solution of sodium chloride. The methylene chloride layer was dried over anhydrous sodium sulfate, and concentrated under reduced pressure.

The residue was purified on a column of 1.5 g of silica gel to give 23 mg of the title compound which showed a UV absorption at an Rf of 0.69 in thin-layer chromatography developed with benzene/-

acetone (1/1).

The resulting compound had the following physicochemical properties.

NMR ($CDCl_3$, TMS), δ (ppm):

0.95 (3H, s, $\underline{CH_3}$-C-$CH_3$),
1.02 (3H, s, $CH_3$-C-$\overline{CH_3}$),
1.40 (3H, s, $\underline{CH_3}$-C(O O)-$\overline{CH_3}$),
1.43 (3H, s, $CH_3$-C(O O)-$\underline{CH_3}$),
2.10 (3H, d, J=7.5Hz, $\underline{CH_3}$-CH=C),
2.39 (2H, t, J=6.5Hz, N-$CH_2$-$\underline{CH_2}$-CO),
3.10-4.95 (10H, m, C-4$H_2$, S-$\overline{CH_2}$-$CH_2$-N, N-$\underline{CH_2}$-$CH_2$-CO, $CH_2$-O),
4.02 (1H, s, O-CH-CO),
4.85 (1H, t, J=9.0Hz, C-5H),
5.43 (2H, s, -$\underline{CH}$-Ar),
6.13 (1H, q, J=7.5Hz, $CH_3$-C$\underline{H}$=C),
6.57 (1H, br, NH),
6.90 (1H, br, NH),
7.58 (2H, d, J=8.0Hz, Ar$\underline{H}$),
8.19 (2H, d, J=8.0Hz, Ar$\underline{H}$).

UV ($CHCl_3$) $\lambda_{max}$: 267 nm (ε =15000).

IR ($CHCl_3$) $\nu_{max}$ $cm^{-1}$:
1780 (β-lactam), 1740 (ester), 1665 (amide), 1320 (sulfone).

<u>Example 14</u>

The following compounds in accordance with this invention were prepared from various known compounds (starting materials for S-oxidation) in accordance with the methods known in Examples 1 to 13 above.

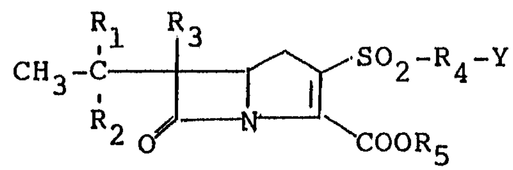

(I)

| Material to be submitted to S-oxidation (in place of $-SO_2-$ above) | $R_5$ | $R_4$ | Y | $R_2$ $R_1$ | $R_3$ |
|---|---|---|---|---|---|
| -S- | 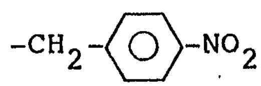 | $-CH_2-CH_2-$ | $-NHCOCH_3$ | $CH_3-CH(R)-$ <br> $OCOCH_3$ | H |
| " | 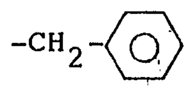 | " | " | $CH_3-CH(S)-$ <br> $OCOCH_2C$ | " |
| " | 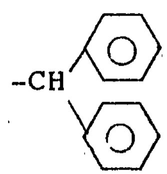 | -CH=CH- | $-NHCOCH_3$ | $CH_3-CH(R)-$ <br> 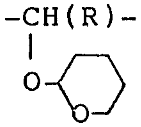 | " |
| " | $-CH_2OCOC(CH_3)_3$ | " | " | $CH_3-CH(S)-$ <br> $OCH_2OCH_3$ | " |

- to be continued -

| Material to be submitted to S-oxidation (in place of $-SO_2-$ above) | $R_5$ | $R_4$ | Y | $R_2$ $R_1$ | $R_3$ |
|---|---|---|---|---|---|
| -S- | $-CH_2OCOC(CH_3)_3$ | $-CH_2-CH_2-$ | $-NHCOCH_3$ | $(CH_3)_2-C-$<br>OH | H |
| " | 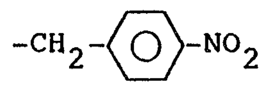 | " | " | " | " |
| -SO- | " | -CH=CH- | " | " | " |
| " | " | $-CH_2-CH_2$ | " | $CH_3-C=$<br>$H_2COCOCH_3$ | |
| " | " | -CH=CH- | " | " | |
| " | " | " | " | $(CH_3)_2C=$ | |
| -S- | " | " | " | $CH_3-C=$<br>$H_2COSO_2CH_3$ | |

Example 15

Production of pivaloyloxymethyl-6-ethyl-3-phenylthio-7-oxo-1-azabicyclo[3.2.0]hept-2-ene-2-carboxylate:-

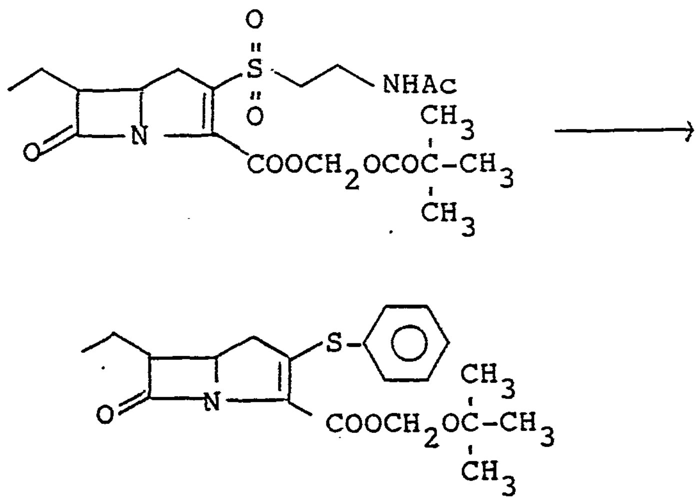

43 mg of PS-5·pivaloyloxymethyl ester·sulfone was dissolved in 4.3 ml of anhydrous dimethylformamide. The solution was cooled to -50°C, and in an atmosphere of nitrogen stream, 21 µl of triethylamine and 13 µl of thiophenol were added. The mixture was reacted at the above temperature for 1 hour.

The reaction mixture was poured into 200 ml of benzene, and washed with 100 ml of 0.01M phosphate buffer (pH 6.8), 100 ml of 0.01M phosphate buffer (pH 8.4) and 100 ml of 0.01M phosphate bufer (pH 6.8). The organic layer was dried over anhydrous sodium sulfate, and concentrated under reduced pressure.

The residue was purified on a column of 4.5 g of silica gel to give 21 mg of a 3-position substituted product which showed an Rf value of 0.38 in thin-layer chromatography developed with benzene/acetone (20/1).

The resulting compound had the following

physicochemical properties.

NMR ($CDCl_3$) δ ppm from TMS:

0.97 (3H, t, J=7.5Hz, $CH_3-CH_2-$),
1.22 (9H, s, $C(CH_3)_3$),
1.73 (2H, m, $CH_3-CH_2-$),
2.62 (2H, d, J=9.0Hz, C-4$H_2$),
2.97 (1H, ddd, J=3.0Hz, J=9.0Hz, J=6.0Hz, C-6H),
3.75 (1H, dt, J=9.0Hz, J=3.0Hz, C-5H),
5.84 (1H, d, J=6.0Hz, O-C(H)(H)-OCO),
5.93 (1H, d, J=6.0Hz, O-C(H)(H)-OCO),
7.38 (5H, m, S-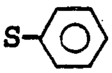).

Example 16

Production of 3-cyclohexylthio-6-ethyl-7-oxo-1-azabicyclo[3.2.0]hept-2-ene-2-carboxylic acid, pNB ester:-

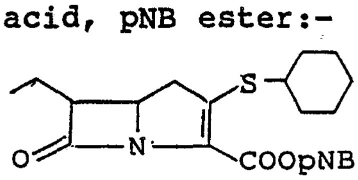

With stirring, 106 l of cyclohexanethiol and 111 l of triethylamine, cooled to -45°C, were added to a solution of 300 mg of a sulfone compound of PS-5 pNB in 40 ml of dimethylformamide. The mixture was stirred at the same temperature for 40 minutes. Then, the reaction mixture was poured into 200 ml of benzene and washed with four 150 ml portions of 0.1M phosphate buffer (pH 6.8). The combined aqueous layers were extracted with three 70 ml portions of benzene. The combined organic layers were washed with two 70 ml portions of the same buffer as above, dried over anhydrous sodium sulfate, and concentrated under

reduced pressure. The resulting yellow oily product was purified by silica gel column chromatography (30 g, 2.6 x 12 cm, benzene/acetone=40:1) to give 174 mg (yield 60%) of the desired product as a yellow powder.

Rf: 0.43 (benzene/acetone=20/1).

$[\alpha]_D^{22}$: +42.6 (c=1.0, THF).

UV $\lambda_{max}^{THF}$ nm ($\varepsilon$): 325 (15600), 270 (12800).

IR $\nu_{max}^{CHCH_3}$ $cm^{-1}$: 1770 (β-lactam CO), 1695 (ester CO).

NMR ($CDCl_3$)δ:

1.08 (3H, t, J=7.5Hz, $CH_2\underline{CH_3}$),
1.2-2.1 (12H, m, $\underline{CH_2}CH_3$, cyclohexyl $CH_2$X5),
2.8-3.2 (4H, m, C-$\overline{4H_2}$, C-6H, SCH),
3.94 (1H, dt, J=9.0Hz, J=3.0Hz, C-5H),
5.18 (1H, d, J=14Hz, ArC$\underline{H}$·H),
5.50 (1H, d, J=14Hz, ArCH·$\underline{H}$),
7.62 (2H, d, J=9.0Hz, ArH),
8.18 (2H, d, J=9.0Hz, ArH).

Mass (m/z): 430 ($M^+$), 360 ($M^+$-Et-CH=C=O).

This compound was obtained also from OA-6129A·pNB ester sulfone by the same reaction.

Example 17

Production of benzyl 3-n-butylthio-6-isopropyl-7-oxo-1-azabicyclo[3.2.0]hept-2-ene-2-carboxylate:-

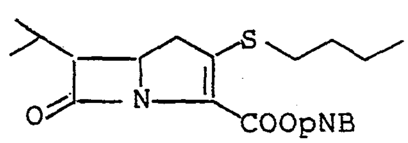

20 mg of PS-6-·benzyl ester·sulfone obtained by reacting PS-6-benzyl ester in the same way as in the production of the sulfone compound of PS-5 pNB ester was dissolved in 10 ml of dry dimethylformamide.

The solution was cooled to -65°C. To the cooled solution was added 1 ml of a dry dimethylformamide solution of 4.9 mg of n-butanethiol sodium salt was added dropwise. After the addition, the mixture was reacted at -65°C for 30 minutes. The reaction mixture was diluted with 50 ml of benzene, washed with three 50 ml portions of 0.1M phosphate buffer (pH, 6.8), and dried over anhydrous sodium sulfate. The solvent was then distilled off under reduced pressure. The residue was dissolved in a small amount of benzene, and charged onto a silica gel column (1 g, 0.8 x 3.4 cm), and the column was developed with benzene-acetone (80:1). The eluates were then charged onto a column (12 x 26.4 cm) of 30 g of Biabeads S-X3 swollen with benzene to give 3.7 mg of the title compound as a white powder (yield 22.1%).

UV $\lambda_{max}^{THF}$: 322 nm

IR $\nu_{max}^{HCl_3}$ $cm^{-1}$: 1780 (β-lactam CO), 1690 (ester CO).

NMR δ ppm ($CDCl_3$):
0.80-1.20 (9H, m, $-CH(C\underline{H_3})_2$, $-CH_2C\underline{H_3}$),
1.20-2.00 (4H, m, 2X-$C\underline{H_2}$-),
2.70-3.60 (6H, m, 2X-$C\underline{H}$-, 2X-$CH_2$-),
3.86-4.18 (1H, m, C-5H),
5.31 (1H, d, J=12Hz, -<u>CH</u>, HAr),
5.47 (1H, d, J=12Hz, -CH, <u>H</u>Ar),
7.45 (5H, s, ArH).

MS m/e: 373 ($M^+$).

Example 18

Production of sodium 3-n-butyl-6-isopropyl-7-oxo-1-azabicyclo[3.2.0]hept-2-ene-2-carboxylate:-

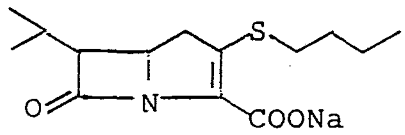

30 mg of benzyl-3-n-butyl-6-isopropyl-7-oxo-1-azabicyclo-[3.2.0]hept-2-ene-2-carboxylate was dissolved in 3 ml of dioxane, and 7 mg of platinum oxide was added, followed by further addition of 1.5 ml of 0.1M phosphae buffer (pH 8.4). This suspension was hydrogenated at room temperature for 10 hours under a hydrogen pressure of 7 kg/$cm^2$ in a Paar apparatus. After the reaction, the reaction mixture was filtered, and then the catalyst removed was washed with 25 ml of 0.01M phosphate bubber. The filtrate and the washing were combined, buffered with 0.01M phosphate buffer, and adsorbed on a column (1.1 x 20.0 cm) of QAE-Sephadex A-25 kept at a low temperature. The column was eluted with 0 to 0.2M sodium chloride by a concentration gradient method (using 100 ml of 0.01M phosphate buffer obtained by adding 0.2M sodium chloride to 100 ml of 0.01M phosphate buffer). 0.1M phosphate buffers had been added in an amount of 0.5 ml each to fraction tubes, and the weight of one fraction was adjusted to 5 g.

Active fractions were determined by an assay plate of <u>Comamonas terrigena</u>, and a saturated aqueous solution of sodium chloride was added so that the final salt concentration became 3%. The mixture was adsorbed on a column (1.1 x 20.0 cm) of Diaion HP-20. The column was washed with water and eluted with 20% acetone-water. 50 µl was taken from each of the eluted fractions (one fraction weighing 5 g), and diluted with 1 ml of water. The ultraviolet spectrum (260-320 nm) of the diluted solution was measured, and those fractions which showed an absorption maximum near 300 nm were collected, and lyophilized for a day

and night (amount yielded 8.3 mg).

UV $\lambda_{max}$ nm ($\varepsilon$): 300 (7800),
(in 0.01M phosphate buffer)

IR $\nu_{max}^{KBr}$ $cm^{-1}$: 1750 ($\beta$-lactam CO),
1600 (carboxylate).

NMR, $\delta$ ppmm ($D_2O$):
0.92 (3H, t, J=8Hz, $-CH_2-CH_3$),
1.06 (6H, dd, J=8Hz, $-CH-(CH_3)_2$),
1.20-2.00 (5H, m, 2X-$CH_2$-, -CH-),
2.50-3.50 (5H, m, 2X-$CH_2$-, -CH-),
4.00 (1H, dt, J=9Hz, J=3Hz, C-5H).

Example 19

Production of pNB ester of 3-(2-hydroxyethylthio)-6-ethyl-7-oxo-1-azabicyclo[3.2.0]hept-2-ene-2-carboxylic acid:-

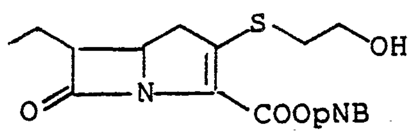

58 µl of TEA and 39 µl of 2-mercaptoethanol were added at -45°C with stirring to a solution of 208 mg of the sulfone compound of PS-5·pNB ester in 50 ml of dimethylformamide. The mixture was stirred at the above temperature. After 40 minutes, the reaction mixture was added to 200 ml of benzene, and washed with four 150 ml portions of 0.1M phosphate buffer (pH 6.8). The solvent was dried over anhydrous sodium sulfate, and evaporated under reduced pressure. Thus, a colorless oily producrt was obtained. This residue was subjected to column chromatography (2.6 x 12 cm) using silica gel (30 g), and the column was eluted with benzene/acetone (10:1, v/v) to give 149.2 mg (85%) of the title compound as a colorless powder.

IR $\nu_{max}^{CHCl_3}$ $cm^{-1}$: 3450 (OH),

1790 (β-lactam C=O),
1700 (ester C=O).

UV $\lambda_{max}^{THF}$ nm(ε): 320 (10400), 270 (10600).

$[\alpha]_D^{22}$: +33.2° (c 1.0 in THF).

NMR δ($CDCl_3$):
1.04 (3H, t, J=7.5Hz, $CH_2\underline{CH_3}$),
1.75-2.05 (2H, m, $\underline{CH_2}CH_3$),
2.85-3.00 (5H, m, 2X$\underline{CH_3}$, CH),
3.70-4.10 (3H, m, C-5$\underline{H}$, -$\underline{CH_2}$OH),
5.18 (1H, d, J=14Hz, ArC$\underline{H}$H),
5.49 (1H, d, J=14Hz, ArCH$\underline{H}$),
7.80 (2H, d, J=9.0Hz, Ar$\underline{H}$),
8.16(2H, d, J=9.0Hz, Ar$\underline{H}$).

MS (m/e): 392 ($M^+$), 322 ($M^+$-EtCH=C=O),
304 ($M^+$-EtCH=C=O-$CH_2$O).

Example 20

Production of sodium 3-(2-hydroxyethylthio)-6-ethyl-7-oxo-1-azebicyclo[3.2.0]hept-2-ene-2-carboxylate:-

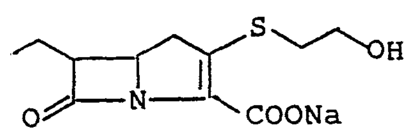

20 mg of p-nitrobenzyl-3-(2-hydroxyethylthio)-6-ethyl-7-oxo-1-azabicyclo[3.2.0]hept-2-ene-2-carboxylate was dissolved in a mixed solvent consisting of 2 ml of dioxane and 1.5 ml of 0.1M phosphate buffer (pH 8.4), and 20 mg of platinum oxide was added. It was hydrogenated at room temperature for 4 hours in a Paar apparatus (hydrogen pressure 4 kg/$cm^2$). After the reaction, the catalyst was removed by filtration. The filtrate was adsorbed on a column (1.1 x 20 cm) of QAE-Sephadex A-25 previously treated with 0.01M phosphate buffer, and the column was eluted with 0-0.4M sodium chloride by a linear

concentration gradient elution method (using 100 ml of a 0.01M phosphate buffer and 100 ml of 0.01M phosphate buffer containing 0.4M sodium chloride). From the eluates, those fractions which showed an absorption maximum at 30 nm in their ultraviolet absorption spectra were collected, and the salt concentration of the solution was adjusted to 3% by sodium chloride. The solution was then adsorbed on a column (1.1 x 20 cm) of Diaion HP-20. The column was eluted with 0-30% acetone by a linear concentration gradient elution method (using 100 ml of deionized water and 100 ml of 30% aqueous acetone solution). Those fractions which showed a maximum absorption at 300 nm in thier ultraviolet absorption spectra were collected and lyophilized to give 5.6 mg (39.4%) of the title compound.

UV $\nu_{max}^{THF}$ nm(ε): 3000 (7302).

NMR δ($D_2O$):

1.00 (3H, t, J=7Hz, $CH_2CH_3$),

1.60-2.00 (2H, m, $CH_2CH_3$),

2.80-3.40 (5H, m, 2X$CH_2$, CH),

3.74 (2H, t, J=6Hz, $CH_2OH$),

4.00 (1H, dt, J=9Hz, J=3Hz, C-5H).

Example 21

Production of p-nitrobenzyl 3-(2-N,N-dimethylaminoethylthio)-6-ethyl-7-oxo-1-azabicyclo[3.2.0]hept-2-ene-2-carboxylate:-

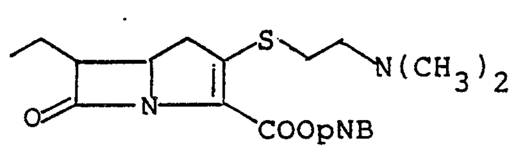

225 mg (0.335 mole) of isopropylidene·OA-6129A.pNB sulfone was dissolved in 70 ml of dry dimethylformamide. The solution was cooled to -60°C, and with stirring, 107 μl of TEA was added. Furthermore, 54.7 mg (0.387 mmole) of N,N-dimethylcysteamine hydrochloride was added. The reaction was carried out for 30 minutes. The reaction mixture was poured into 300 ml of benzene, and washed with two 150 ml portions of 0.1M phosphate buffer (pH 8.5) and 200 ml of a saturated aqueous solution of sodium chloride. The organic layer was dried over anhydrous sodium sulfate and distilled under reduced pressure to remove benzene. Thus, a pale yellow oily product was obtained.

This product was charged onto a column (1.2 x 80 cm) of Sephadex LH 20 swollen and filled with acetone. The column was developed with acetone. The desired product was searched by thin-layer chromatography, and fractions containing the desired product were collected and distilled under reduced pressure to remove the solvent. There was obtained 10 mg (yield 75%) of the desired compound.

IR $\nu_{max}^{CHCl_3}$ $cm^{-1}$: 1779 (β-lactam C=O), 1705 (ester C=O).

UV $\lambda_{max}^{THF}$ nm(ε): 322 (12100), 270 (10700).

$[\alpha]_D^{22}$ +38.5° (c 1.0, THF).

NMR δ($CDCl_3$):
1.04 (3H, t, J=7.5Hz, $-CH_2CH_3$),
1.70-2.00 (2H, m, $-CH_2CH_3$),
2.28 (6H, s, $-N(CH_3)_2$).
2.44-3.40 (7H, m, C-6H, C-4$H_2$, N-$CH_2CH_2$-S),
3.98 (1H, dt, J=4Hz, J=9Hz, C-5H),
5.24 (1H, d, J=14Hz, -CHHAr),
5.54 (1H, d, J-14Hz, -CHHAr),
7.68 (2H, d, J=9Hz, $-CH_2$ArH),
8.24 (2H, d, J=9Hz, $-CH_2$ArH).

Mass (m/e): 419 ($M^+$), 404 ($M^+-CH_3$), 349 ($M^+$-EtCH=C=O).

Example 22

Production of 3-(2-N,N-dimethylaminoethylthio-6-ethyl-7-oxo-1-azabicyclo[3.2.0]hept-2-ene-2-carboxylic acid:-

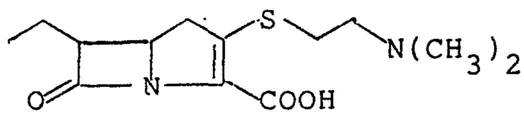

120 mg of p-nitrobenzyl 3-(2-N,N-dimethylaminoethylthio)-6-ethyl-7-oxo-1-azabicyclo[3.2.0]-hept-2-ene-2-carboxylate was dissolved in a 50% aqueous solution of dioxane, and 120 mg of platinum oxide was added. It was reacted at room temperature for 4 hours in a Paar apparatus (hydrogen pressure 4 kg/cm$^2$). The catalyst was filtered off and the filtrate and washing were combined and concentrated under reduced pressure to 3 ml. The concentrate was chargd onto a column (1.0 x 20 cm of Dowex 50W (x8) Na-type. The column was eluted with water, and fractions each weighing 5 g were obtained. Those fractions which showed an ultraviolet absorption at 297 nm were collected and lyophilized to give 32 mg (yield 39%) of the desird product.

UV $\lambda_{max}^{H_2O}$ nm($\varepsilon$): 297 (7026).

NMR $\delta$ ($D_2O$):

1.01 (3H, t, J=7.5Hz, $-CH_2\underline{CH_3}$),
1.60-2.00 (2H, m, $-\underline{CH_2}CH_3$),
2.65 (6H, s, $N(\underline{CH_3})_2$),
3.00-3.50 (7H, m, C-4$\underline{H_2}$, C-6H, $-S-\underline{CH_2}\underline{CH_2}N<$),
4.04 (1H, dt, J=3Hz, J=9Hz, C-5$\underline{H}$).

Example 23

Production of pivaloyloxymethyl 6-ethyl-3-(imidazol-4-yl)methylthio-7-oxo-1-azabicyclo[3.2.0]hept-2-ene-2-caboxylate:-

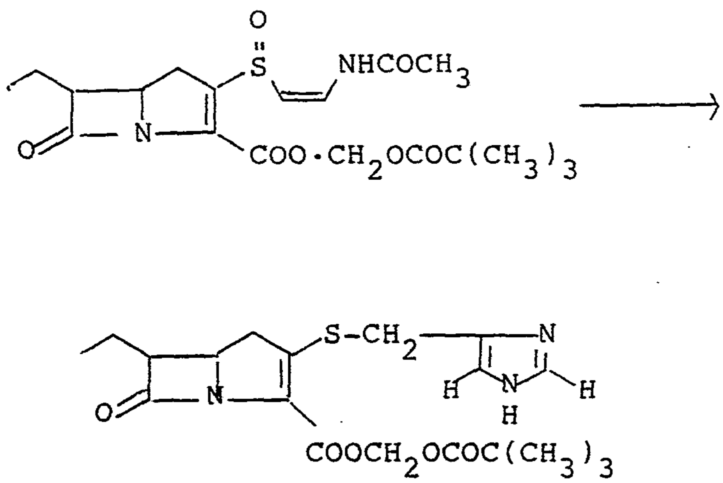

59 mg of pivaloyloxymethyl 3-N-acetyldehydrocysteaminyl-6-ethyl-7-oxo-1-azabicyclo[3.2.0]hept-2-ene-2-carboxylate sulfone synthesized in Example 4 was dissolved in 20 ml of dry dimethylformamide. The solution was cooled to -40°C, and with stirring, 1.13 ml of a dimethylformamide solution of sodium hydrosulfide ($NaSH \cdot xH_2O$, abt. 70%) in a concentration of 10 mg/ml was added. The mixture was stirred at the above temperature for 30 minutes, followed by addition of 73 µl of triethylamine and further a solution of 30.0 mg of 4-chloromethylimidazole in 1.5 ml of dimethylformamide. The mixture was stirred at the same temperature for 40 minutes. The reaction mixture was poured into 120 ml of benzene, and washed with three 70 ml portions of 0.1M phosphate buffer (pH 8.40). The organic phase was dried over anhydrous sodium sulfate, concentrated under reduced pressure, and dried in vacuum. 60 mg of the residue was charged into a column (1.2 x 84 cm) of Sephadex LH-20, and the

column was eluted with acetone. The fractions were examined by thin-layer chromatography, and those fractions which contained the desired product and showed an Rf of 0.18 in TLC developed with benzene/acetone=1:3 were collected and dried under reduced pressure to give 44 mg of the desired product as a yellow oily product.

UV $\lambda_{max}^{THF}$ nm: 325.0, 264.0.

IR $\nu_{max}^{CHCl_2}$ $cm^{-1}$: 1773 (β-lactam C=O), 1750 (ester C=O), 1720 (ester C=O shoulder).

NMR, δ ppm ($CDCl_3$):

1.01 (3H, t, J=7.5Hz, $-CH_2-C\underline{H_3}$),
1.20 (9H, s, $-C(C\underline{H_3})_3$),
1.53-2.03 (2H, m, $-C\underline{H_2}-CH_3$),
3.04 (1H, dd, J=9.0Hz, J=18.0Hz, C-4$\underline{H}$),
2.85-3.30 (1H, m, C-6$\underline{H}$),
3.40 (1H, dd, J=9.0Hz, J=18.0Hz, C-4$\underline{H}$),
3.87 (1H, dt, J=3.0Hz, J=9.0Hz, C-5$\underline{H}$),
4.03 (2H, s, $-S-C\underline{H_2}-$),
5.80 (2H, d, J=5.5Hz, $-CO-O-C\underline{H}\cdot H-O-$),
5.92 (2H, d, J=5.5Hz, $-CO-O-CH\cdot \underline{H}-O-$),
5.50-6.0 (1H, broad, >N$\underline{H}$),
6.97 (1H, s, Imd-5'$\underline{H}$),
7.60 (1H, s, Imd-2'$\underline{H}$).

Mass (m/z) 407 ($M^+$), 337 ($M^+$-EtCH=C=O).

Example 24

Production of p-nitrobenzyl 6-(1-acetoxy)-ethyl-3-phenylthio-7-oxo-1-azabicyclo-[3.2.0]hept-2-ene-2-carboxylate:-

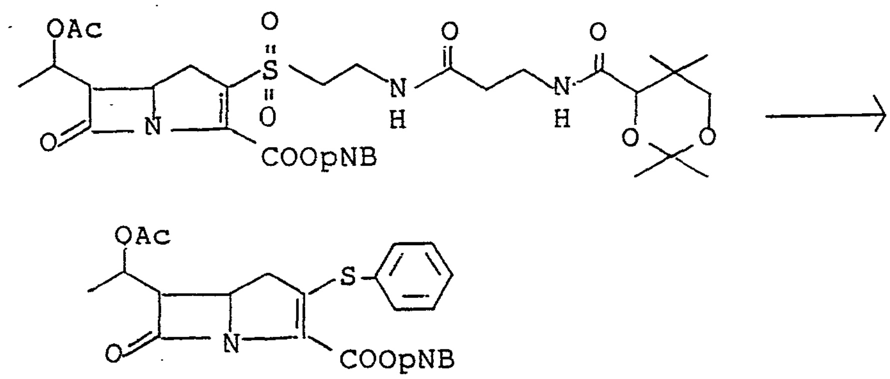

47 mg of 8-O-acetyl·isopropylidene·OA-6129$B_2$ pNB sulfone was dissolved in 20 ml of anhydrous dimethylformamide. The solution was cooled to -45°C, and in a stream of nitrogen, 14 μl of triethylamine and 8 μl of thiophenol were added. The reaction was carried out at the above temperature for 10 minutes.

The reaction mixture was poured into 100 ml of benzene, and washed with 50 ml of 0.01M phosphate buffer (pH 6.8), 50 ml of 0.01M phosphate buffer (pH 8.4) and 50 ml of 0.01M phosphate buffer (pH 6.8). The organic layer was dried over anhydrous sodium sulfate, and concentrated under reduced pressure. The residue was chromatograhed on a column of 4 g of silica gel to obtain the 3-position substituted product which showed a UV absorption at an Rf value of 0.34 in thin-layer chromatography developed with benzene/acetone (18:1).

The resulting compound had the following physicochemical properties.

Specific rotation $[\alpha]_D^{24}$: 10.9° (C=1.0, $CHCl_3$).

UV ($CHCl_3$) $\lambda_{max}$ nm(ε): 320 (11100), 270 (9800).

IR ($CHCl_3$) $\nu_{max}$ $cm^{1}$: 1782 (β-lactam), 1740, 1705 (ester).

NMR ($CDCl_3$, TMS) δ(ppm):

1.36 (3H, d, J=6.5Hz, CH-$C\underline{H_3}$),

2.03 (3H, s, $COCH_3$),
2.66 (2H, d, J=9.0Hz, C-$4H_2$),
3.28 (1H, dd, J=3.0Hz, J=4.5Hz, C-6H),
3.92 (1H, dt, J=3.0Hz, J=9.0Hz, C-5H),
4.95-5.40 (2H, m, C<u>H</u>-$CH_3$, C<u>H</u>H-Ar),
5.50 (1H, d, J=14.0Hz, CH<u>H</u>-Ar),
7.20-7.57 (5H, m, S-phe),
7.60 (2H, J=9.0Hz, Ar<u>H</u>),
8.15 (2H, d, J=9.0Hz, Ar<u>H</u>).

MS (EI):
m/z 482 (M), 440 (M-$CH_2$=CO),
364 (M-$CH_3$-CHOAc-CH=CO).

What we claim is:

1. A process for producing a beta-lactam compound of the general formula

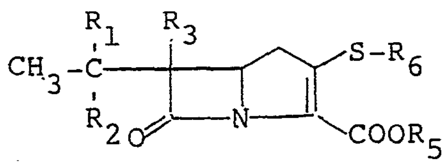 (III)

wherein R represents a hydrogen atom or a methyl group, $R_2$ represents a hydrogen atom, a hydroxyl group which may be protected or a hydroxymethyl group which may be protected, $R_3$ represents a hydrogen atom, or together with $R_1$ forms a single bond, $R_5$ represents an ester residue and $R_6$ represents a hydrogen atom or a monovalent organic group,

which comprises reacting a compound of the general formula

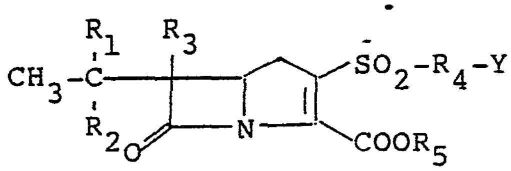 (I)

wherein $R_1$, $R_2$, $R_3$ and $R_5$ are as defined above, $R_4$ represents $-CH_2-CH_2-$ or $-CH=CH-$, and Y represents a protected amino group,

with a thiol compound of the formula

$R_6$-SH (II)

or its reactive derivative.

2. The process of claim 1 wherein the reaction is carried out in an inert polar solvent.

3. The process of claim 1 wherein the solvent is N,N-dimethylformamide.

4. The process of claim 1 wherein the reaction is

carried out at room temperature or at lower temperatures.

5. The process of claim 1 wherein the reaction is carried out at a temperature in the range of from about-30$^{o}$C to about -50$^{o}$C.

6. The process of claim 1 wherein the compound of formula (I) is reacted with the thiol compound of formula (II) in the presence of a base.

7. The process of claim 6 wherein the base is used in an amount of at least 1 equivalent per mole of the thiol compound.

8. The process of claim 1 wherein the reactive derivative of the thiol compound is a thiolate compound of the formula

$R_6SM$ (II')

wherein M represents an alkali metal, and $R_6$ has the meanings defined in claim 1.

9. The process of claim 1 wherein the thiol compound of formula (II) or its reactive derivative is used in an amount of at least 1 mole per mole of the compound of formula (I).

10. The process of claim 1 wherein the thiol compound of formula (II) or its reactive derivative is used in an amount of 1.1 to 1.5 moles per mole of the compound of formula (I).

11. The process of claim 1 wherein the protecting group in the optionally protected hydroxyl group or the optionally protected hydroxymethyl group is selected from the class consisting of lower alkyl groups, substituted or unsubstituted aralkyl groups, lower alkoxyalkyl groups, acyl groups, alkoxycarbonyl groups and organic sulfonyl groups.

12. The process of claim 1 wherein the protecting group in the protected amino group is selected from the class consisting of (a) substituted or

unsubstituted aralkyl groups, (b) groups of the formula $-COR_9$ in which $R_9$ represents a hydrogen atom, an alkyl group, a cycloalkyl group, a substituted or unsubstituted aryl group or a substituted or unsubstituted aralkyl group, or groups of the formula

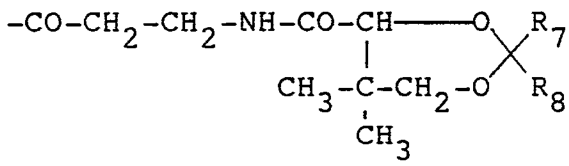

in which $R_7$ and $R_8$ each represent a hydrogen atom, a lower alkyl group or a phenyl group, or $R_7$ and $R_8$ together represent an alkylene group with 4 or 5 carbon atoms, (c) groups of formula $-COOR_{10}$ in which $R_{10}$ represents an alkyl group, a cycloalkyl group or a substituted or unsubstituted aralkyl group, (d) groups of the formula $-SO_2-R_{11}$ in which $R_{11}$ represents a lower alkyl group or a substituted or unsubstituted aryl group, (e) groups of the formula

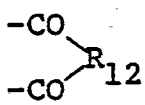

in which $R_{12}$ represents a lower alkylene group, a lower alkenylene group or a phenylene group, and (f) a trimethylsilyl group and a 4,4-dimethyl-1-oxo-2-hexen-3-yl group.

13. The process of claim 1 wherein $R_6$ is a monovalent organic group having up to 30 carbon atoms, preferably up to 25 carbon atoms.

14. The process of claim 13 wherein the monovalent organic groups is selected from the class consisting of (i) substituted or unsubstituted alkyl, alkenyl and alkynyl groups, (ii) cycloalkyl groups, (iii) cycloalkyl-alkyl groups, (iv) substituted or unsubstituted aryl groups, (v) substituted or unsubstituted aralkyl groups, (vi) heterocyclic groups, and (vii) heterocyclic-alkyl groups.

15. The process of claim 1 wherein $R_5$ in formula (I) is an ester residue easily splittable by hydrolysis or hydrogenolysis, the resulting compound of formula (III) is subjected to saponification or hydrogenolysis in a customary manner to form a compound of formula (III) in which $R_5$ is a hydrogen atom, or its salt.

16. The process of claim 1 wherein $R_5$ is selected from the class consisting of pivaloyloxymethyl, phthalidyl, methylthiomethyl, benzyl, benzhydryl, trityl, p-nitrobenzyl, 2,4-dinitrobenzyl and p-chlorobenzyl groups.

17. The process of claim 1 wherein $R_6$ is selected from the class consisting of lower alkyl groups, $C_5$-$C_6$ cycloalkyl groups, substituted lower alkyl groups and 5- or 6-membered heterocyclic groups containing 1 to 4 N, O or S atoms as hetero atoms.

18. The process of claim 1 wherein the compound of formula (I) is prepared by S-oxidation of the compound of the formula

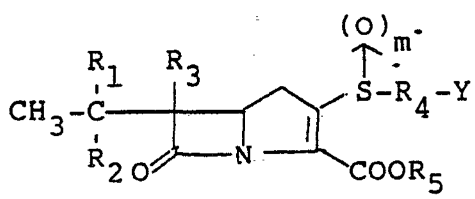

(VI)

wherein m is 0 or 1, and

$R_1$, $R_2$, $R_3$, $R_4$, $R_5$ and Y have the meanings defined in claim 1.

19. A beta-lactam compound of the general formula

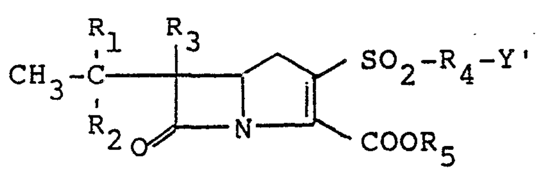

(I-1)

wherein

$R_1$ represents a hydrogen atom or a methyl group,

0105227

$R_2$ represents a hydrogen atom, a hydroxyl group which may be protected, or a hydroxymethyl group which may be protected,

$R_3$ represents a hydrogen atom, or together with $R_1$ forms a single bond,

$R_4$ represents $-CH_2-CH_2-$ or $-CH=CH-$,

$R_5$ represents an ester residue, and

Y' represents a protected amino group,

provided that when both $R_1$ and $R_3$ are hydrogen atoms, $R_2$ is a hydroxyl group which may be protected and $R_4$ is $-CH_2-CH_2-$, then Y' represents a gruop of the formula

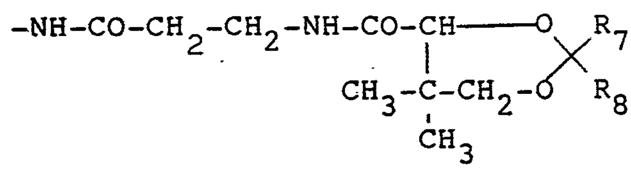

wherein each of $R_7$ and $R_8$ represents a hydrogen atom, a lower alkyl group or a phenyl group, or $R_7$ and $R_8$ together represent an alkylene group having 4 or 5 carbon atoms.

20. The compound of claim 19 wherein $R_2$ is a hydrogen atom, a hydroxyl group, an acetyloxy group, a chloroacetyloxy group, a tetrahydropyranyloxy group, a methoxymethoxy group, an acetyloxymethyl group or a mesyloxymethyl group.

21. The compound of claim 19 wherein $R_5$ is a pivaloyloxymethyl, phthalidyl, methylthiomethyl, benzyl, benzhydryl, trityl, p-nitrobenzyl, 2,4-dinitrobenzyl or p-chlorobenzyl group.

22. The compound of claim 19 wherein Y' is an acetylamino group.

0105227

European Patent Office

# EUROPEAN SEARCH REPORT

Application number

EP 83 10 8896

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | CHEMICAL ABSTRACTS, vol. 96, no. 21, 24th May 1982, page 691, no. 181065a, Columbus, Ohio, US & JP - A - 81 156 281 (SANRAKU-OCEAN CO., LTD.) 02-12-1981 * Abstract * --- | 1-5,8 | C 07 D 487/04 // (C 07 D 487/04 C 07 D 209/00 C 07 D 205/00 ) |
| P,Y | EP-A-0 060 612 (BEECHAM) * Claims * & JP - A - 57 146 778 (published 10-09-1982) --- | 1-22 | |
| P,A | EP-A-0 068 485 (SANRAKU-OCEAN) * Claims * ----- | 19-22 | |
| | | | TECHNICAL FIELDS SEARCHED (Int. Cl. 3) |
| | | | C 07 D 487/00 |

The present search report has been drawn up for all claims

| Place of search | Date of completion of the search | Examiner |
|---|---|---|
| THE HAGUE | 28-11-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document